# EUROPÄISCHE PATENTSCHRIFT

(11) **EP 3 864 016 B1**
(45) Veröffentlichungstag und Bekanntmachung des Hinweises auf die Patenterteilung: **14.09.2022**
(21) Anmeldenummer: 19780274.7
(22) Anmeldetag: 09.10.2019
(51) Int. Cl.: C07D 471/04, C07D 487/04, C07F 3/06

(54) **VERFAHREN ZUR HERSTELLUNG SUBSTITUIERTER IMIDAZOLDERIVATE**
PROCESS FOR PREPARING SUBSTITUTED IMIDAZOLE DERIVATIVES
PROCÉDÉ DE PRÉPARATION DE DÉRIVÉS D'IMIDAZOLE SUBSTITUÉS

(30) Priorität: 11.10.2018 EP 18199890
(43) Veröffentlichungstag der Anmeldung: 18.08.2021
(73) Patentinhaber: Bayer Aktiengesellschaft, 51373 Leverkusen (DE)
(72) Erfinder: WILLOT, Matthieu, 40215 Düsseldorf (DE); FISCHER, Rüdiger, 50259 Pulheim (DE); HAGER, Dominik, 40789 Monheim (DE); HOFFMEISTER, Laura, 40593 Düsseldorf (DE); MOSRIN, Marc, 40789 Monheim am Rhein (DE); WILCKE, David, 40237 Düsseldorf (DE)
(74) Vertreter: BIP Patents
(86) Internationale Anmeldenummer: PCT/EP2019/077307
(87) Internationale Veröffentlichungsnummer: WO 2020/074560

(56) Entgegenhaltungen:
- EP-A1- 2 662 365
- WO-A1-2018/130437
- WO-A1-2018/130443
- COLLINS I ET AL: "A Convenient Synthesis of Highly Substituted 2-Pyridones", TETRAHEDRON LETTERS, ELSEVIER, AMSTERDAM, NL, Bd. 40, Nr. 21, 21. Mai 1999 (1999-05-21), Seiten 4069-4072, XP004164614, ISSN: 0040-4039, DOI: 10.1016/S0040-4039(99)00651-6
- MARC MOSRIN ET AL: "TMPZnCl?LiCl: A New Active Selective Base for the Directed Zincation of Sensitive Aromatics and Heteroaromatics", ORGANIC LETTERS, Bd. 11, Nr. 8, 16. April 2009 (2009-04-16) , Seiten 1837-1840, XP055019579, ISSN: 1523-7060, DOI: 10.1021/ol900342a
- CHRISTOS I. STATHAKIS ET AL: "TMPZnOPiv-LiCl: A New Base for the Preparation of Air-Stable Solid Zinc Pivalates of Sensitive Aromatics and Heteroaromatics", ORGANIC LETTERS, Bd. 15, Nr. 6, 15. März 2013 (2013-03-15), Seiten 1302-1305, XP055522219, US ISSN: 1523-7060, DOI: 10.1021/ol400242r
- MARK L. HLAVINKA ET AL: "Zn(tmp) 2 : A Versatile Base for the Selective Functionalization of C-H Bonds", ORGANOMETALLICS, Bd. 26, Nr. 17, 1. August 2007 (2007-08-01), Seiten 4105-4108, XP055522203, US ISSN: 0276-7333, DOI: 10.1021/om700475t

## Beschreibung

Die vorliegende Erfindung betrifft ein Verfahren zur Herstellung von substituierten Imidazolderivaten der Formel (II) 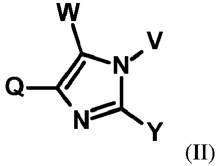 ausgehend von Verbindungen der Formel (I) 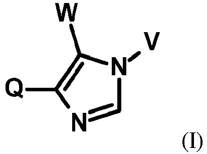 über Zwischenstufen der Formel (IVa) oder (IVb) 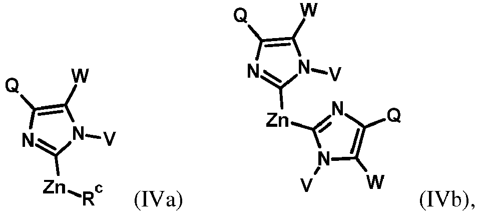 in denen die in den Formeln (I), (II), (IVa) und (IVb) angegebenen Strukturelemente die nachstehend angegebenen Bedeutungen haben.

Ferner betrifft die Erfindung die Zwischenstufen der Formeln (IVa) und (IVb).

Substituierte Imidazolderivate der Formel (II) haben große technische Bedeutung für die pharmazeutische und die agrochemische Industrie und sind beispielsweise wichtige Zwischenprodukte unter anderem bei der Herstellung von Verbindungen, die beispielsweise als Pestizide wirksam sind.

Entsprechende Imidazole zur Verwendung als Pestizide und Verfahren zu deren Herstellung sind beispielsweise beschrieben in WO 2013/180193, WO 2016/039444, WO 2018/130437 und WO 2018/130443.

Die im Stand der Technik beschriebenen Herstellungsverfahren umfassen allerdings Methoden, die aus industrieller Sicht wirtschaftlich nicht zu realisieren sind und/oder andere Nachteile aufweisen.

Insbesondere stellt die regioselektive Einführung des Substituenten Y an Verbindungen der Formel (I) eine große Herausforderung dar. Besonders schwierig gestaltet sich dies durch die beiden chemisch sehr ähnlichen Ringsysteme die in den Verbindungen der Formel (I) aneinander gekoppelt sind und durch die Präsenz anderer sauren Protonen in den Verbindungen der Formel (I). In der Literatur ist die Metallierung und folgende Halogenierung von solchen Strukturen auf dem Imidazol Ring bisher nicht beschrieben.

Nachteilig sind insbesondere bei den üblicherweise verwendeten Lithium- und Magnesiumbasen die geringen chemischen Ausbeuten, die Durchführung bei sehr niedrigen Temperaturen sowie die schwierige Regio- und Chemo-Selektivität der Deprotoniereung aufgrund der hohen Reaktivität von diesen Reagenzien. Manchmal ist eine Transmetallierung mit Zinksalzen wie zum Beispiel Zinkchlorid notwendig, um weitere selektive Reaktionen wie Negishi-Kreuzkupplungen durchzuführen, wie in Organic Letters 2008, 10, 2497-2500 beschrieben. Die Herstellung ist daher sehr teuer (viele Salze entstehen) und nicht für großtechnische kommerzielle Verfahren geeignet.

Im Hinblick auf die vorstehend geschilderten Nachteile besteht dringend Bedarf für ein vereinfachtes, technisch und ökonomisch durchführbares Verfahren zur Herstellung von substituierten Imidazolderivaten, insbesondere der Formel (II). Die mit diesem angestrebten Verfahren erhältlichen substituierten Imidazolderivate sollen dabei vorzugsweise mit guter Ausbeute, hoher Reinheit und in ökonomischer Weise erhalten werden.

Verfahren zur Herstellung von substituierten Imidazopyridinen und deren Vorstufen unter Verwendung von Zink-metallorganischen Basen sind beschrieben in WO 2018/007224, WO 2018/033448, WO 2018/050515 und WO 2018/141955.

Überraschenderweise wurde gefunden, dass in einem Verfahren unter Verwendung einer Zink-metallorganischen Base auch substituierten Imidazolderivate der Formel (II) vorteilhaft, insbesondere auch mit hoher Regio- und Chemoselektivität und guter Ausbeute hergestellt werden können.

Gegenstand der vorliegenden Erfindung ist demnach ein Verfahren zur Herstellung von Verbindungen der Formel (II) 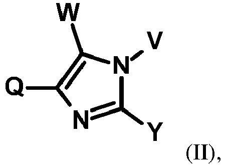 in welcher (Ausgestaltung 1)
Q für ein Strukturelement 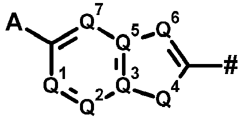
steht, wobei das Zeichen # die Bindung zum Rest des Moleküls anzeigt und
Q¹ für N oder CR⁶ steht,
Q² für N oder CR⁶ steht,
Q³ für N oder C steht,
Q⁴ für O, S, N, CR⁶ oder NR⁷ steht,
Q⁵ für N oder C steht,
Q⁶ für N oder CH steht und
Q⁷ für N oder CH steht,
wobei maximal fünf der Variablen Q¹, Q², Q³, Q⁴, Q⁵, Q⁶ und Q⁷ gleichzeitig für Stickstoff stehen und Q³ und Q⁵ nicht gleichzeitig für N stehen und
R⁶ für Wasserstoff, (C₁-C₄)Alkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht und
R⁷ für (C₁-C₄)Alkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(Ci-C₄)alkyl, (C₂-C₄)Halogenalkenyloxy-(Ci-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
A für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonyl steht, oder
A für -O-CF₂-O- steht und gemeinsam mit Q¹ und dem Kohlenstoffatom, an das es gebunden ist, einen fünfgliedrigen Ring bildet, wobei Q¹ für Kohlenstoff steht,
W für Halogen oder S(O)ₙR⁸ steht, wobei
R⁸ für (C₁-C₆)Alkyl, (C₁-C₆)Cyanoalkyl, (C₁-C₆)Alkoxy-(C₁-C₆)alkyl, (C₁-C₆)Halogenalkyl, (C₂-C₆)Alkenyl, (C₂-C₆)Halogenalkenyl, (C₂-C₆)Alkinyl, (C₂-C₆)Halogenalkinyl oder (C₃-C₈)Cycloalkyl steht und
n für 0, 1 oder 2 steht,
V für (C₁-C₆)-Alkyl, (C₁-C₆)Halogenalkyl, (C₁-C₆)Cyanoalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₈)Cycloalkyl-(C₃-C₈)Cycloalkyl, (C₁-C₆)Alkyl-(C₃-C₈)Cycloalkyl, Halogen(C₃-C₈)cycloalkyl, Cyano(C₃-C₈)cycloalkyl, (C₁-C₆)Alkylthio-(C₁-C₆)alkyl, (C₁-C₆)Halogenaₗkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl oder (C₁-C₆)Halogenalkoxycarbonyl-(C₁-C₆)alkyl steht und
Y für Halogen, Cyano, Nitro, (C₁-C₄)Alkyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, SCN, (C₁-C₄)Alkylcarbonyl, (C₁-C₄)Halogenalkylcarbonyl, (C₁-C₄)Alkoxycarbonyl, (C₁-C₄)Halogenalkoxycarbonyl, Aminocarbonyl, (C₁-C₄)Alkylaminocarbonyl, Di-(C₁-C₄)alkyl-aminocarbonyl, (C₁-C₄)Halogenalkylaminocarbonyl, (C₃-C₆)Cycloalkylaminocarbonyl, Aminothiocarbonyl, (C₁-C₄)Alkylaminothiocarbonyl, Di-(C₁-C₄)alkylaminothiocarbonyl, (C₁-C₄)Halogenalkylaminothiocarbonyl, (C₃-C₆)Cycloalkylaminothiocarbonyl, Amino, (C₁-C₄)Alkylamino, (C₁-C₄)Halogenalkylamino, Di-(C₁-C₄)Alkylamino, (C₃-C₆)Cycloalkylamino, (C₁-C₄)Alkylsulfonylamino, (C₁-C₄)Alkylcarbonylamino, (C₁-C₄)Halogenalkylcarbonylamino, (C₁-C₄)Alkylcarbonyl-(C₁-C₄)alkyl-amino, (C₁-C₄)Halogenalkylcarbonyl-(C₁-C₄)alkyl-amino, (C₃-C₆)Cycloalkylcarbonylamino, (C₃-C₆)Cycloalkylcarbonyl-(C₁-C₄)alkyl-amino, (C₁-C₄)Alkylthiocarbonylamino, (C₁-C₄)Halogenalkylthiocarbonylamino, (C₁-C₄)Alkylthiocarbonyl-(C₁-C₄)alkyl-amino, (C₁-C₄)Halogenalkylthiocarbonyl-(C₁-C₄)alkyl-amino, (C₃-C₆)Cycloalkylthiocarbonylamino, (C₃-C₆)Cycloalkylthiocarbonyl-(C₁-C₄)alkyl-amino, (C₂-C₄)Alkenyl, (C₂-C₄)Halogenalkenyl, (C₂-C₄)Cyanoalkenyl, (C₃-C₆)Cycloalkyl-(C₂)alkenyl, (C₂-C₄)Alkinyl oder (C₂-C₄)Halogenalkinyl steht,
   oder für jeweils gegebenenfalls einfach oder mehrfach, gleich oder verschieden substituiertes (C₃-C₆)Cycloalkyl oder (C₅-C₆)Cycloalkenyl steht, wobei als Substituenten jeweils in Frage kommen: (C₃-C₆)Cycloalkyl, (C₁-C₄)Alkyl, (C₁-C₄)Halogenalkyl, (C₁-C₄)Alkoxy, (C₁-C₄)Halogenalkoxy, Aminocarbonyl, Aminothiocarbonyl, Halogen oder Cyano,
   oder für jeweils gegebenenfalls einfach oder mehrfach, gleich oder verschieden substituiertes Aryl oder Hetaryl steht, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, SF₅, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Halogenalkyl-(C₃-C₆)cycloalkyl, Halogen-(C₃-C₆)cycloalkyl, Cyano-(C₃-C₆)cycloalkyl, (C₁-C₄)Alkyl, (C₁-C₄)Halogenalkyl, (C₁-C₄)Cyanoalkyl, (C₁-C₄)Hydroxyalkyl, Hydroxycarbonyl-(C₁-C₄)-alkoxy, (C₁-C₄)Alkoxycarbonyl-(C₁-C₄)alkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₃-C₆)Cycloalkyl-(C₂)alkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxycarbonyl-(C₁-C₄)alkoxy, (C₁-C₄)Alkoxy-(C₁-C₄)alkoxy, (C₁-C₄)Alkoxyimino, (C₁-C₄)Halogenalkoxyimino, (C₁-C₄)Alkylthio, (C₁-C₄)Halogenalkylthio, (C₁-C₄)Alkoxy-(C₁-C₄)alkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkoxy-(C₁-C₄)alkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkoxy-(C₁-C₄)alkylsulfonyl, (C₁-C₄)Alkylsulfonyl-(C₁-C₄)alkyl, (C₁-C₄)Alkylsulfonyloxy, (C₁-C₄)Halogenalkylsulfonyloxy, (C₁-C₄)Alkylcarbonyl, (C₁-C₄)Halogenalkylcarbonyl, (C₁-C₄)Alkylcarbonyloxy, (C₁-C₄)Alkoxycarbonyl, (C₁-C₄)Halogenalkoxycarbonyl, Aminocarbonyl, (C₁-C₄)Alkylaminocarbonyl, (C₁-C₄)Halogenalkylaminocarbonyl, Di-(C₁-C₄)alkyl-aminocarbonyl, (C₂-C₄)Alkenylaminocarbonyl, Di-(C₂-C₄)-alkenylaminocarbonyl, (C₃-C₆)Cycloalkylaminocarbonyl, (C₁-C₄)Alkylsulfonylamino, (C₁-C₄)Alkylamino, Di-(C₁-C₄)Alkylamino, (C₁-C₄)Halogenalkylamino, (C₃-C₆)Cycloalkylamino, Aminosulfonyl, (C₁-C₄)Alkylaminosulfonyl, Di-(C₁-C₄)alkylaminosulfonyl, (C₁-C₄)Alkylsulfoximino, Aminothiocarbonyl, (C₁-C₄)Alkylaminothiocarbonyl, Di-(C₁-C₄)alkylaminothiocarbonyl, (C₁-C₄)Halogenalkylaminothiocarbonyl, (C₃-C₆)Cycloalkylaminothiocarbonyl, (C₁-C₄)Alkylcarbonylamino, (C₁-C₄)Halogenalkylcarbonylamino, (C₁-C₄)Alkylcarbonyl-(C₁-C₄)alkyl-amino, (C₁-C₄)Halogenalkylcarbonyl-(C₁-C₄)alkyl-amino, (C₃-C₆)Cycloalkylcarbonylamino, (C₃-C₆)Cycloalkylcarbonyl-(C₁-C₄)alkyl-amino, (C₁-C₄)Alkylthiocarbonylamino, (C₁-C₄)Halogenalkylthiocarbonylamino, (C₁-C₄)Alkylthiocarbonyl-(C₁-C₄)alkyl-amino, (C₁-C₄)Halogenalkylthiocarbonyl-(C₁-C₄)alkyl-amino, (C₃-C₆)Cycloalkylthiocarbonylamino, (C₃-C₆)Cycloalkylthiocarbonyl-(C₁-C₄)alkyl-amino, Hetaryl, Oxo-Hetaryl, Halogen-Hetaryl, Halogen-Oxo-Hetaryl, Cyano-Hetaryl, Cyano-Oxo-Hetaryl, (C₁-C₄)Halogenalkyl-Hetaryl oder (C₁-C₄)Halogenalkyl-Oxo-Hetaryl,
dadurch gekennzeichnet, dass in einem ersten Verfahrensschritt a) eine Verbindung der Formel (I) 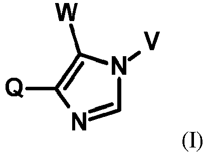
in welcher Q, V und W jeweils die oben genannten Bedeutungen haben,
mit einer Zink-metallorganischen Base der Struktur (NR^{a}R^{b})-Zn-R^{c} oder (NR^{a}R^{b})₂-Zn, in welcher
R^{c} für Halogen oder -O-Pivaloyl steht und
R^{a} und R^{b} gemeinsam eine -(CH₂)₄-, -(CH₂)₅- oder -(CH₂)₂O(CH₂)₂-Gruppe bilden, wobei jede dieser Gruppen optional durch 1,2, 3 oder 4 R^{d}-Radikale substituiert sein kann und R^{d} ausgewählt ist aus der Gruppe bestehend aus Methyl, Ethyl, n-Propyl und i-Propyl,
zu einer Verbindung der Formel (IVa) oder der Formel (IVb) umgesetzt wird, 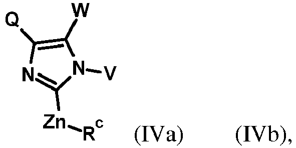
in welcher Q, V, W und R^{c} jeweils die oben genannten Bedeutungen haben,
und diese Verbindung der Formel (IVa) oder (IVb) in einem zweiten Verfahrensschritt b) mit einer Verbindung der Struktur Y-X, in welcher X für Halogen steht und Y die oben genannte Bedeutung hat, zur Verbindung der Formel (II) umgesetzt wird.

X steht dabei bevorzugt für Chlor, Brom, Iod oder Fluor, besonders bevorzugt für Brom oder Iod und ganz besonders bevorzugt für Iod.

Die Verbindungen der Formel (IVa) und (IVb) können auch mit Salzen komplexiert vorliegen, wobei es sich bei den Salzen bevorzugt um Alkali- oder Erdalkalihalogenide handelt, vorzugsweise um Lithiumchlorid und/oder Magnesiumchlorid und besonders bevorzugt um Lithiumchlorid.

Vorzugsweise stehen maximal vier der Variablen Q¹, Q², Q³, Q⁴, Q⁵, Q⁶ und Q⁷ gleichzeitig für Stickstoff. Unter Stickstoff werden hierbei N und/oder NR⁷ verstanden.

Bevorzugte Bedeutungen der in den vorstehend erwähnten Formeln (I), (II), (IVa) und (IVb) des erfindungsgemäßen Verfahrens aufgeführten Reste Q, V, W und R^{c} werden im Folgenden erläutert, wobei die Zink-metallorganische Base weiter unten noch genau beschrieben wird, so dass die bevorzugten Ausgestaltungen der Base dort angegeben sind.

### (Ausgestaltung 2)

- Q: steht bevorzugt für ein Strukturelement aus der Reihe Q¹ bis Q14, 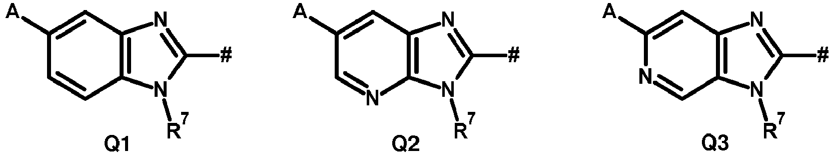 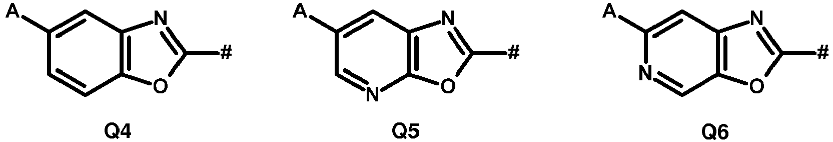 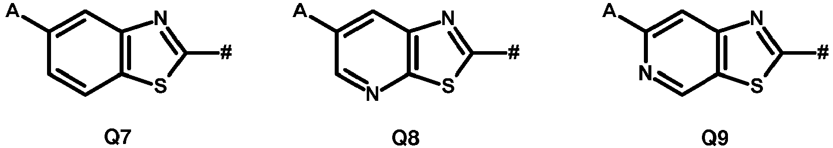 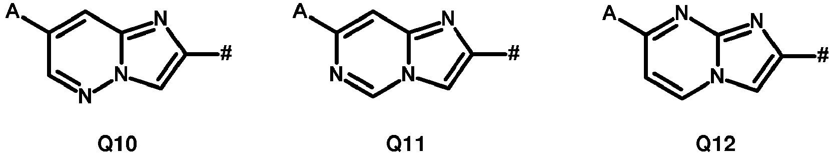 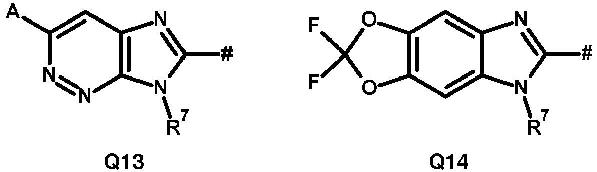 , wobei
- R⁷: bevorzugt für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht und
- A: bevorzugt für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl steht,
- W: steht bevorzugt für Halogen oder S(O)ₙR⁸, wobei
- R⁸: bevorzugt für (C₁-C₆)Alkyl, (C₁-C₆)Cyanoalkyl, (C₁-C₆)Alkoxy-(C₁-C₆)alkyl, (C₁-C₆)Halogenalkyl, (C₂-C₆)Alkenyl, (C₂-C₆)Halogenalkenyl, (C₂-C₆)Alkinyl, (C₂-C6)Halogenalkinyl oder (C₃-C₈)Cycloalkyl steht und
- n: bevorzugt für 0, 1 oder 2 steht,
- R^{c}: steht bevorzugt für Halogen, insbesondere Chlor, Brom oder Iod,
- V: steht bevorzugt für (C₁-C₆)-Alkyl, C₁-C₆)Cyanoalkyl, (C₁-C₆)Alkoxy-(C₁-C₆)alkyl, (C₁-C₆)Halogenalkyl, (C₂-C₆)Alkenyl, (C₂-C₆)Halogenalkenyl, (C₂-C₆)Alkinyl, (C₂-C6)Halogenalkinyl oder (C₃-C₈)Cycloalkyl und
- Y: steht bevorzugt für Halogen, Cyano, (C₁-C₄)Alkyl, (C₁-C₄)Halogenalkyl, (C₁-C₄)Alkoxy, (C₁-C₄)Halogenalkoxy, Aminocarbonyl, (C₁-C₄)Alkylaminocarbonyl, Di-(C₁-C₄)alkyl-aminocarbonyl, (C₁-C₄)Halogenalkylaminocarbonyl, (C₃-C₆)Cycloalkylaminocarbonyl, Amino, (C₁-C₄)Alkylamino, (C₁-C₄)Halogenalkylamino, Di-(C₁-C₄)Alkylamino, (C₃-C6)Cycloalkylamino, (C₁-C₄)Alkylsulfonylamino, (C₁-C₄)Alkylcarbonylamino, (C₁-C₄)Halogenalkylcarbonylamino, (C₁-C₄)Alkylcarbonyl-(C₁-C₂)alkyl-amino, (C₁-C₄)Halogenalkylcarbonyl-(C₁-C₂)alkyl-amino, (C₃-C₆)Cycloalkylcarbonylamino, (C₃-C₆)Cycloalkylcarbonyl-(C₁-C₂)alkyl-amino, (C₂-C₄)Alkenyl, (C₂-C₄)Halogenalkenyl, (C₂-C₄)Cyanoalkenyl oder (C₃-C₆)Cycloalkyl-(C₂)alkenyl,
oder für jeweils gegebenenfalls einfach oder mehrfach, gleich oder verschieden substituiertes (C₃-C₆)Cycloalkyl oder (C₅-C₆)Cycloalkenyl, wobei als Substituenten jeweils in Frage kommen: (C₃-C₆)Cycloalkyl, (C₁-C₄)Alkyl, (C₁-C₄)Halogenalkyl, (C₁-C₄)Alkoxy, (C₁-C₄)Halogenalkoxy, Halogen oder Cyano,
oder für jeweils gegebenenfalls einfach oder mehrfach, gleich oder verschieden substituiertes Phenyl, Pyridyl, Pyrimidyl, Pyridazinyl, Thiophenyl, Furanyl, Pyrazolyl, Pyrrolyl, Thiazolyl, Oxazolyl oder Imidazolyl, wobei als Substituenten jeweils in Frage kommen: Cyano, Halogen, Nitro, Acetyl, Hydroxy, Amino, SF₅-, (C₃-C₆)Cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₂)alkyl, (C₂-C₄)Alkenyl, (C₂-C₄)Halogenalkenyl, (C₂-C₄)Cyanoalkenyl, (C₃-C₆)Cycloalkyl-(C₂)alkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₂)alkoxy, (C₁-C₄)Alkoxyimino, (C₁-C₄)Halogenalkoxyimino, (C₁-C₄)Alkylthio, (C₁-C₄)Halogenalkylthio, (C₁-C₄)Alkylthio-(C₁-C₂)alkyl, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Halogenalkylsulfonyloxy, (C₁-C₄)Alkylcarbonyl, (C₁-C₄)Halogenalkylcarbonyl, Aminocarbonyl, (C₁-C₄)Alkylaminocarbonyl, (C₁-C₄)Halogenalkylaminocarbonyl, Di-(C₁-C₄)alkyl-aminocarbonyl, (C₃-C₆)Cycloalkylaminocarbonyl, Aminothiocarbonyl, (C₁-C₄)Alkylaminothiocarbonyl, Di-(C₁-C₄)alkylaminothiocarbonyl, (C₁-C₄)Halogenalkylaminothiocarbonyl, (C₃-C₆)Cycloalkylaminothiocarbonyl, (C₁-C₄)Alkylsulfonylamino, (C₁-C₄)Alkylamino, Di-(C₁-C₄)Alkylamino, (C₁-C₄)Halogenalkylamino, (C₃-C₆)Cycloalkylamino, Aminosulfonyl, (C₁-C₄)Alkylaminosulfonyl, Di-(C₁-C₄)alkyl-aminosulfonyl, (C₁-C₄)Alkylcarbonylamino, (C₁-C₄)Halogenalkylcarbonylamino, (C₁-C₄)Alkylcarbonyl-(C₁-C₂)alkyl-amino, (C₁-C₂)Halogenalkylcarbonyl-(C₁-C₂)alkyl-amino, (C₃-C₆)Cycloalkylcarbonylamino, (C₃-C₆)Cycloalkylcarbonyl-(C₁-C₂)alkyl-amino, (C₁-C₄)Alkylthiocarbonylamino, (C₁-C₄)Halogenalkylthiocarbonylamino, (C₁-C₄)Alkylthiocarbonyl-(C₁-C₂)alkyl-amino, (C₁-C₄)Halogenalkylthiocarbonyl-(C₁-C₂)alkyl-amino, (C₃-C₆)Cycloalkylthiocarbonylamino oder (C₃-C₆)Cycloalkylthiocarbonyl-(C₁-C₂)alkyl-amino.

### (Ausgestaltung 3)

- Q: steht besonders bevorzugt für ein Strukturelement aus der Reihe Q2, Q3, Q4, Q10, Q11, Q13 oder Q14, wobei
- R⁷: besonders bevorzugt für (C₁-C₄)Alkyl oder (C₁-C₄)Alkoxy-(C₁-C₄)alkyl steht und
- A: besonders bevorzugt für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl steht,
- W: steht besonders bevorzugt für Halogen oder S(O)ₙR⁸, wobei
- R⁸: besonders bevorzugt für Methyl, Ethyl, n-Propyl oder iso-Propyl steht und
- n: besonders bevorzugt für 0,1 oder 2 steht,
- R^{c}: steht besonders bevorzugt für Chlor,
- V: steht besonders bevorzugt für Methyl, Ethyl, n-Propyl oder iso-Propyl und
- Y: steht besonders bevorzugt für Brom, Iod, Cyano, Ethenyl, Cyclopropylethenyl, i-Propenyl, Cyclopropylethinyl, Methyl, Ethyl, Isopropyl, Cyclopropylethyl, Methoxycarbonyl, Trifluorethylaminocarbonyl, Aminocarbonyl, Methylaminocarbonyl, Dimethylaminocarbonyl, Ethylaminocarbonyl, Aminothiocarbonyl, Methylaminothiocarbonyl, Dimethylaminothiocarbonyl,
oder für jeweils gegebenenfalls einfach oder zweifach, gleich oder verschieden substituiertes Cyclopropyl, Cyclobutyl, Cyclopentyl, Cyclohexyl, Cyclopentenyl oder Cyclohexenyl, wobei als Substituenten jeweils in Frage kommen: Methyl, Ethyl, n-Propyl, i-Propyl, cyclo-Propyl, Difluormethyl, Trifluormethyl, Cyano, Fluor oder Chlor,
oder für jeweils gegebenenfalls einfach, zweifach oder dreifach, gleich oder verschieden substituiertes Phenyl, Pyridin-2-yl, Pyridin-3-yl, Pyridin-4-yl, Pyrimidin-5-yl, Pyridazin-3-yl, Pyridazin-4-yl, Thien-2-yl, Thien-3-yl, 1,3-Thiazol-5-yl, 1H-Imidazol-1-yl, 1H-Imidazol-2-yl, 1H-Imidazol-5-yl, 1H-Pyrazol-1-yl, 1H-Pyrazol-3-yl, 1H-Pyrazol-4-yl, 1H-Pyrazol-5-yl, 1H-Pyrrol-1-yl, 1H-Pyrrol-2-yl, 1H-Pyrrol-3-yl oder 1-Cyclohexenyl, wobei als Substituenten jeweils in Frage kommen: Cyano, Fluor, Chlor, Methyl, Cyclopropyl, Cyanomethyl, Cyanoisopropyl, Cyanocylopropyl, Trifluormethyl, Trifluorethyl oder Aminocarbonyl.

### (Ausgestaltung 4)

- Q: steht ganz besonders bevorzugt für das Strukturelement Q3 oder Q13, wobei
- R⁷: ganz besonders bevorzugt für Methyl oder Ethyl, insbesondere für Methyl steht und
- A: ganz besonders bevorzugt für Trifluormethyl oder Pentafluorethyl steht,
- W: steht ganz besonders bevorzugt für S(O)ₙR⁸, wobei
- R⁸: ganz besonders bevorzugt für Ethyl steht und
- n: ganz besonders bevorzugt für 0 oder 2 steht,
- R^{c}: steht ganz besonders bevorzugt für Chlor,
- V: steht ganz besonders bevorzugt für Methyl oder Ethyl, insbesondere für Methyl und
- Y: steht ganz besonders bevorzugt für Brom, 5-Cyanpyridin-2-yl oder 6-Chlorpyridazin-3-yl.

Die vorstehend aufgeführten Restedefinitionen bzw. Erläuterungen gelten sowohl für die Endprodukte und Zwischenprodukte als auch für die Ausgangsprodukte entsprechend. Diese Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Erfindungsgemäß ganz besonders bevorzugt werden solche Verbindungen, in welchen eine Kombination der vorstehend als ganz besonders bevorzugt aufgeführten Bedeutungen vorliegt.

In einer weiteren bevorzugten Ausführungsform der Erfindung steht Q für Q¹ und R⁷, A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 5).

In einer weiteren bevorzugten Ausführungsform der Erfindung steht Q für Q2 und R⁷, A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 6).

In einer weiteren bevorzugten Ausführungsform der Erfindung steht Q für Q3 und R⁷, A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 7).

In einer weiteren bevorzugten Ausführungsform der Erfindung steht Q für Q4 und A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 8).

In einer weiteren bevorzugten Ausführungsform der Erfindung steht Q für Q5 und A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 9).

In einer weiteren bevorzugten Ausführungsform der Erfindung steht Q für Q6 und A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 10).

In einer weiteren bevorzugten Ausführungsform der Erfindung steht Q für Q7 und A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 11).

In einer weiteren bevorzugten Ausführungsform der Erfindung steht Q für Q8 und A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 12).

In einer weiteren bevorzugten Ausführungsform der Erfindung steht Q für Q9 und A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 13).

In einer weiteren bevorzugten Ausführungsform der Erfindung steht Q für Q10 und A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 14).

In einer weiteren bevorzugten Ausführungsform der Erfindung steht Q für Q11 und A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 15).

In einer weiteren bevorzugten Ausführungsform der Erfindung steht Q für Q12 und A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 16).

In einer weiteren bevorzugten Ausführungsform der Erfindung steht Q für Q13 und R⁷, A, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 17).

In einer weiteren bevorzugten Ausführungsform der Erfindung steht Q für Q14 und R⁷, W, R⁸, n, R^{c}, V und Y haben die in Ausgestaltung 1 oder die in Ausgestaltung 2 oder die in Ausgestaltung 3 oder die in Ausgestaltung 4 angegebenen Bedeutungen (Ausgestaltung 18).

In einer besonders bevorzugten Ausführungsform der Erfindung steht Q für Q2, Q3, Q4, Q10, Q11, Q13 oder Q14 und R⁷, A, W, R⁸, n, R^{c}, V und Y haben die in Ausgestaltung 1 oder die in Ausgestaltung 2 oder die in Ausgestaltung 4 angegebenen Bedeutungen (Ausgestaltung 19).

In einer besonders bevorzugten Ausführungsform der Erfindung steht Q für Q3 oder Q13 und R⁷, A, W, R⁸, n, R^{c}, V und Y haben die in Ausgestaltung 1 oder die in Ausgestaltung 2 oder die in Ausgestaltung 3 angegebenen Bedeutungen (Ausgestaltung 20).

Vorteilhafterweise lassen sich die substituierten Imidazolderivate der Formel (II) mit dem erfindungsgemäßen Verfahren mit guten Ausbeuten und in hoher Reinheit herstellen. Aufgrund der sehr guten funktionellen Gruppen-Toleranz von Zink-Reagenzien sind Zinkbasen sehr attraktiv. Regio- und chemoselektive Metallierungen von Imidazolderivaten in Gegenwart von stöchiometrischen Mengen von selektiven Basen werden möglich, auch bei erhöhten Temperaturen, ohne dass eine Arin-Eliminierung stattfindet oder empfindliche funktionelle Gruppen angegriffen werden. Die als Zwischenstufe entstandene Zinkverbindung kann anschließend mit verschiedenen Elektrophilen abgefangen werden wie beispielsweise in Organic Letters 2009, 11, 1837-1840 beschrieben. Diese neu substituierten Imidazolderivate können dann als wertvolle Synthone weiter umgesetzt werden. Durch das erfindungegemäße Verfahren werden zudem weitere und/oder flexiblere Derivatisierungen von Edukt und Produkt möglich, ohne Synthesenrouten ständig ändern bzw. anzupassen zu müssen.

Das erfindungsgemäße Verfahren kann anhand des folgenden Schemas (I) erläutert werden:

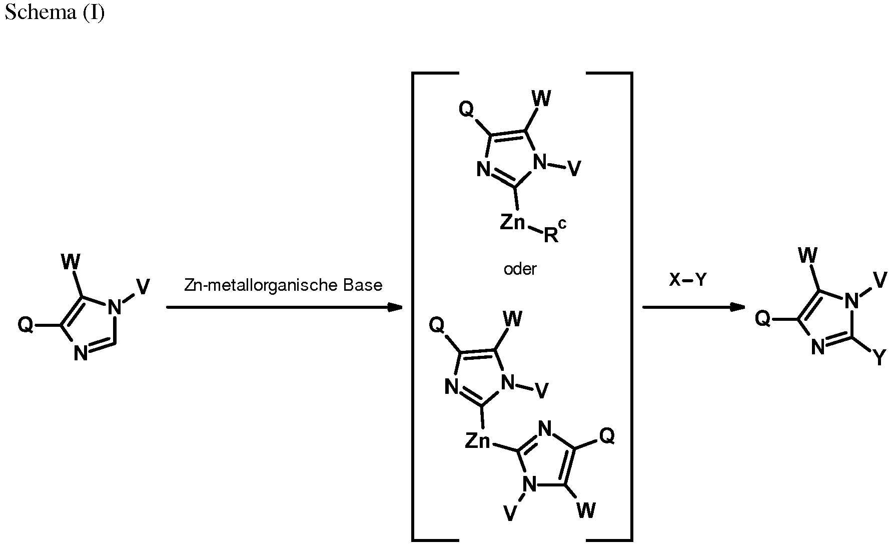

Hierin haben Q, V, W, R^{c}, X und Y die vorstehend angegebenen Bedeutungen. Die in Klammern angegebenen Verbindungen stellen die Zwischenstufe (Formel (IVa) beziehungsweise (IVb)) dar, welche weiter zur Verbindung der Formel (II) umgesetzt wird. Demnach lässt sich das erfindungsgemäße Verfahren in die beiden Verfahrensschritte a) und b) unterteilen, wobei Schritt a) die Umsetzung der Verbindung der Formel (I) zur jeweiligen Zwischenstufe und Schritt b) die weitere Umsetzung der Zwischenstufe zur Verbindung der Formel (II) ist.

### Allgemeine Definitionen

Definitionsgemäß ist, sofern nichts anderes angegeben ist, Halogen ausgewählt aus der Reihe Fluor, Chlor, Brom und Iod.

Der Begriff "Halogenide" beschreibt im Zusammenhang mit der vorliegenden Erfindung Verbindungen zwischen Halogenen und Elementen anderer Gruppen des Periodensystems, wobei salzartige Halogenide (ionische Verbindungen (Salze)), die aufgrund der großen Elektronegativitätsdifferenz zwischen den beteiligten Elementen aus Anionen und Kationen bestehen und durch elektrostatische Wechselwirkungen zusammengehalten werden) oder kovalente Halogenide (kovalente Verbindungen, bei denen der Elektronegativitätsunterschied nicht so groß ist wie bei den vorstehend genannten ionischen Verbindungen, die Bindungen jedoch eine Ladungspolarität aufweisen) vorliegen können, abhängig von der Art der chemischen Bindung. Erfindungsgemäß besonders bevorzugt sind salzartige Halogenide.

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl.

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden.

Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Sofern nicht an anderer Stelle anders definiert, wird unter dem Begriff "Aryl" erfindungsgemäß ein aromatischer Rest mit 6 bis 14 Kohlenstoffatomen, vorzugsweise Phenyl, Naphthyl, Anthryl oder Phenanthrenyl, besonders bevorzugt Phenyl, verstanden.

Sofern nicht an anderer Stelle anders definiert, wird unter dem Begriff "Arylalkyl" eine Kombination von erfindungsgemäß definierten Resten "Aryl" und "Alkyl" verstanden, wobei der Rest im Allgemeinen über die Alkylgrupe gebunden wird, Beispiele hierfür sind Benzyl, Phenylethyl oder α-Methylbenzyl, wobei Benzyl besonders bevorzugt ist.

Sofern nicht an anderer Stelle anders definiert, bedeutet "Hetaryl" bzw "heteroaromatischer Ring" eine mono-, bi- oder tricyclische heterocyclische Gruppe aus C-Atomen und mindestens einem Heteroatom, wobei mindestens ein Zyklus aromatisch ist. Bevorzugt enthält die Hetaryl-Gruppe 3, 4, 5, 6, 7 oder 8 C-Atome. Besonders bevorzugt sind dabei monocyclische Gruppen aus 3, 4, 5, 6, 7 oder 8 C-Atomen und mindestens einem Heteroatom.Insbesondere bevorzugt ist die Hetaryl-Gruppe ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl, Imidazopyridinyl und Indolizinyl.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Der Begriff "Pivaloyl" beschreibt im Zusammenhang mit der vorliegenden Erfindung den deprotonierten Rest der Pivalinsäure (IX) mit der Summenformel (CH₃)₃CCO₂H. 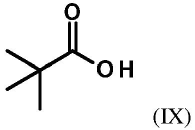 "O-Pivaloyl" bedeutet entsprechend, dass die Bindung des Pivaloylrestes über das deprotonierte Sauerstoffatom der Säuregruppe erfolgt.

Die Synthese von Verbindungen der Formel (I) ist dem Fachmann grundsätzlich bekannt und ist beispielsweise beschrieben in WO 2013/180193, WO 2016/039444, WO 2018/130437 und WO 2018/130443.

Die Umsetzung der Verbindungen der Formel (I) zu Verbindungen der Formel (IVa) oder Formel (IVb) im ersten Verfahrensschritt (Schritt a)) erfolgt in Gegenwart einer Zink-metallorganischen Base der Struktur (NR^{a}R^{b})-Zn-R^{c} oder (NR^{a}R^{b})₂-Zn, in welcher (Ausgestaltung B-1)
R^{c} wie vorstehend (Ausgestaltung 1) definiert ist (daher für Halogen oder -O-Pivaloyl steht),
R^{a} und R^{b} gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R^{d}-Radikale substituiert sein kann und
R^{d} ausgewählt ist aus der Gruppe bestehend aus Methyl, Ethyl, n-Propyl und i-Propyl.

### Bevorzugt ist, dass (Ausgestaltung B-2)

R^{c} wie vorstehend als bevorzugt (Ausgestaltung 2) definiert ist (daher für -O-Pivaloyl, Chlor, Brom oder Iod steht),
R^{a} und R^{b} gemeinsam eine -(CH₂)₅-Gruppe bilden, wobei jede dieser Gruppen optional durch 1, 2, 3 oder 4 R^{d}-Radikale substituiert sein kann und
R^{d} ausgewählt ist aus der Gruppe bestehend aus Methyl und Ethyl.

### Besonders bevorzugt ist, dass (Ausgestaltung B-3)

R^{c} wie vorstehend als besonders bevorzugt (Ausgestaltung 6) definiert ist (daher für Chlor steht) und
R^{a} und R^{b} gemeinsam eine -(CH₂)₅-Gruppe bilden, die durch 4 Methylgruppen substituiert ist.

Die vorstehend aufgeführten Restedefinitionen können untereinander, also auch zwischen den jeweiligen Vorzugsbereichen, beliebig kombiniert werden.

In einer ganz besonders bevorzugten Ausgestaltung der erfindungsgemäßen Base ist das Strukturelement (NR^{a}R^{b}) Tetramethylpiperidin (TMP) gemäß Formel (V).

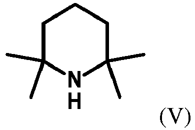

Erfindungsgemäß ganz besonders bevorzugte Zink-metallorganische Basen sind demnach dadurch gekennzeichnet, dass Zink gebunden an TMP vorliegt, insbesondere als Zinkhalogenid und ganz besonders bevorzugt als Zinkchlorid. Derartige Basen weisen folgende Struktur der Formel (VI) auf

### (Ausgestaltung B-4)

(VI) (TMP)ₓ ZnCl₂₋ₓ ,

worin x für die Zahl 1 oder 2 steht. Hierunter wiederum bevorzugt sind Basen mit x=1 (Ausgestaltung B-5) gemäß Formel (VII):

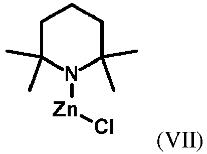

In einer weiteren bevorzugten Ausführungsform des erfindungsgemäßen Verfahrens liegt die metallorganische Base in Verbindung mit Alkali- oder Erdalkalihalogeniden vor. Dies gilt insbesondere für Basen der Formeln (VI) und (VII). Besonders bevorzugte derartige Alkali- oder Erdalkalihalogenide sind Lithiumchlorid und Magnesiumchlorid, ganz besonders bevorzugt ist Lithiumchlorid. Erfindungsgemäß ganz besonders bevorzugte metallorganische Basen sind demnach TMP ZnCl·LiCl oder (TMP)₂ Zn·2LiCl oder (TMP)₂ Zn·2LiCl 2 MgCl₂ (Ausgestaltung B-6). Am meisten bevorzugt ist TMP ZnCl·LiCl (VIII; Ausgestaltung B-7).

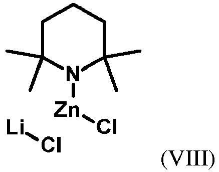

Beispielhaft werden in nachfolgender Tabelle 1 konkrete Kombinationen von Verbindungen der Formeln (I), (II) und (IVa) bzw. (IVb) mit erfindungsgemäßen Basen genannt, die in einem erfindungsgemäßen Verfahren zur Anwendung kommen können. Da in einigen Ausgestaltungen das Strukturelement R^{c} sowohl in der erfindungsgemäßen Base als auch in der Verbindung der Formel (IVa) vorliegt, gilt für R^{c} jeweils die engste Definition.

**Tabelle 1:**

| Nummer | Verbindungen der Formeln (I), (II) und (IVa) bzw. (IVb) | Base gemäß |
|---|---|---|
| 1 | Ausgestaltung 1 | Ausgestaltung B-1 |
| 2 | Ausgestaltung 1 | Ausgestaltung B-2 |
| 3 | Ausgestaltung 1 | Ausgestaltung B-3 |
| 4 | Ausgestaltung 1 | Ausgestaltung B-4 |
| 5 | Ausgestaltung 1 | Ausgestaltung B-5 |
| 6 | Ausgestaltung 1 | Ausgestaltung B-6 |
| 7 | Ausgestaltung 1 | Ausgestaltung B-7 |
| 8 | Ausgestaltung 2 | Ausgestaltung B-1 |
| 9 | Ausgestaltung 2 | Ausgestaltung B-2 |
| 10 | Ausgestaltung 2 | Ausgestaltung B-3 |
| 11 | Ausgestaltung 2 | Ausgestaltung B-4 |
| 12 | Ausgestaltung 2 | Ausgestaltung B-5 |
| 13 | Ausgestaltung 2 | Ausgestaltung B-6 |
| 14 | Ausgestaltung 2 | Ausgestaltung B-7 |
| 15 | Ausgestaltung 3 | Ausgestaltung B-1 |
| 16 | Ausgestaltung 3 | Ausgestaltung B-2 |
| 17 | Ausgestaltung 3 | Ausgestaltung B-3 |
| 18 | Ausgestaltung 3 | Ausgestaltung B-4 |
| 19 | Ausgestaltung 3 | Ausgestaltung B-5 |
| 20 | Ausgestaltung 3 | Ausgestaltung B-6 |
| 21 | Ausgestaltung 3 | Ausgestaltung B-7 |
| 22 | Ausgestaltung 4 | Ausgestaltung B-1 |
| 23 | Ausgestaltung 4 | Ausgestaltung B-2 |
| 24 | Ausgestaltung 4 | Ausgestaltung B-3 |
| 25 | Ausgestaltung 4 | Ausgestaltung B-4 |
| 26 | Ausgestaltung 4 | Ausgestaltung B-5 |
| 27 | Ausgestaltung 4 | Ausgestaltung B-6 |
| 28 | Ausgestaltung 4 | Ausgestaltung B-7 |
| 29 | Ausgestaltung 19 | Ausgestaltung B-1 |
| 30 | Ausgestaltung 19 | Ausgestaltung B-2 |
| 31 | Ausgestaltung 19 | Ausgestaltung B-3 |
| 32 | Ausgestaltung 19 | Ausgestaltung B-4 |
| 33 | Ausgestaltung 19 | Ausgestaltung B-5 |
| 34 | Ausgestaltung 19 | Ausgestaltung B-6 |
| 35 | Ausgestaltung 19 | Ausgestaltung B-7 |
| 36 | Ausgestaltung 20 | Ausgestaltung B-1 |
| 37 | Ausgestaltung 20 | Ausgestaltung B-2 |
| 38 | Ausgestaltung 20 | Ausgestaltung B-3 |
| 39 | Ausgestaltung 20 | Ausgestaltung B-4 |
| 40 | Ausgestaltung 20 | Ausgestaltung B-5 |
| 41 | Ausgestaltung 20 | Ausgestaltung B-6 |
| 42 | Ausgestaltung 20 | Ausgestaltung B-7 |

Vorzugsweise wird die metallorganische Base in dem erfindungsgemäßen Verfahren in einer Gesamtmenge von 0,5 bis 5,0 Äquivalenten, vorzugsweise von 0,8 bis 2,0 Äquivalenten, weiter bevorzugt von 1,0 bis 1,5 Äquivalenten und besonders bevorzugt von 1,0 bis 1,2 Äquivalenten, bezogen auf die Stoffmenge an Verbindung der Formel (I), eingesetzt. Ein Vorteil des erfindungsgemäßen Verfahrens ist es diesbezüglich, dass die metallorganische Base in nahezu stöchiometrischen Mengen eingesetzt werden kann.

In Abhängigkeit davon, ob in der eingesetzten Zink-metallorganischen Base das Strukturelement (NR^{a}R^{b}) einfach oder zweifach vorhanden ist, entstehen Zwischenverbindungen der Formel (IVa) beziehungsweise der Formel (IVb) in Verfahrensschritt a).

Bei erhöhten Temperaturen ist der Umsatz des Verfahrensschrittes a) besonders gut. Die Umsetzung in Verfahrensschritt a) wird daher im Allgemeinen bei einer Temperatur zwischen 0 °C und 110 °C und zunehmend bevorzugt zwischen 20 °C und 100 °C, zwischen 30 °C und 95 °C, zwischen 40 °C und 90 °C, zwischen 60 °C und 85 °C und ganz besonders bevorzugt zwischen 70 °C und 85 °C, beispielsweise bei 80 °C, durchgeführt.

Die Reaktion wird üblicherweise bei Normaldruck durchgeführt, kann aber auch bei erhöhtem bzw. vermindertem Druck durchgeführt werden.

Schritt a) erfolgt im Allgemeinen in einem Zeitraum von 5 min bis 12 h, bevorzugt von 15 min bis 10 h und besonders bevorzugt von 30 min bis 2 h.

Die Umsetzung der Verbindungen der Formel (IVa) beziehungsweise (IVb) zu Verbindungen der Formel (II) im zweiten Verfahrensschritt (Schritt b)) erfolgt in Gegenwart einer Verbindung X-Y, in welcher X und Y jeweils die vorstehend genannten Bedeutungen haben.

### Einführung des Restes Y (Schritt b) für Y =Halogen):

In einer Variante der Erfindung stehen sowohl X als auch Y für Halogen. In diesem Fall handelt es sich bei X-Y um eine Interhalogenverbindung. X und Y müssen dabei nicht notwendigerweise für das gleiche Halogen stehen. Beispielsweise kann X für Iod oder Brom stehen und Y für Chlor, Brom oder Iod. Vorzugsweise handelt es sich bei der Verbindung X-Y aber um ein elementares Halogen, insbesondere F₂, Cl₂, Br₂ oder I₂. Besonders bevorzugt sind I₂ oder Br₂, ganz besonders bevorzugt ist Br₂.

Bevorzugt ist X-Y so gewählt, dass die Reste W und Y für unterschiedliche Halogene stehen, besonders bevorzugt steht für den Fall, dass
W für Fluor steht, Y für Iod, Brom oder Chlor oder
W für Chlor steht, Y für Iod oder Brom oder
W für Brom steht, Y für Iod.

Vorzugsweise wird die Verbindung X-Y in dem erfindungsgemäßen Verfahren in einer Gesamtmenge von 0,5 bis 10 Äquivalenten, vorzugsweise von 0,8 bis 5,0 Äquivalenten, weiter bevorzugt von 1,0 bis 2,5 Äquivalenten und besonders bevorzugt von 1,0 bis 1,5 Äquivalenten, bezogen auf die Stoffmenge an Verbindung der Formel (I), eingesetzt.

Die Umsetzung in Verfahrensschritt b) wird im Allgemeinen bei einer Temperatur zwischen 0 °C und 80 °C und zunehmend bevorzugt zwischen 10 °C und 70 °C, zwischen 15 °C und 60 °C, zwischen 20 °C und 50 °C, zwischen 20 °C und 40 °C und ganz besonders bevorzugt zwischen 20 °C und 35 °C, beispielsweise bei Raumtemperatur bzw. 25 °C, durchgeführt.

Die Reaktion wird üblicherweise bei Normaldruck durchgeführt, kann aber auch bei erhöhtem bzw. vermindertem Druck durchgeführt werden.

Schritt b) erfolgt in dieser Variante der Erfindung im Allgemeinen in einem Zeitraum von 5 min bis 12 h, bevorzugt von 15 min bis 10 h und besonders bevorzugt von 30 min bis 2 h.

*Einführung des Restes Y* (*Schritt b*) *für Y* ≠ *Halogen*)*:*In einer weiteren Variante der Erfindung erfolgt die Umsetzung der Verbindungen der Formel (IVa) oder (IVb) zu Verbindungen der Formel (II) im zweiten Verfahrensschritt (Schritt b)) in Gegenwart einer Verbindung X-Y, in welcher Y die Bedeutung gemäß einer der Ausgestaltungen 1 bis 4 hat, aber nicht für Halogen steht und X bevorzugt für Chlor, Brom, Iod oder Fluor (Ausgestaltung (C-1), besonders bevorzugt für Brom oder Iod (C-2) und ganz besonders bevorzugt für Iod (C-3) steht.

Beispielhaft werden in nachfolgender Tabelle 2 Verbindungen X-Y aufgeführt, die in einem erfindungsgemäßen Verfahren zur Anwendung kommen können.

**Tabelle 2:**

| Nummer | Y | X |
|---|---|---|
| 1 | Ausgestaltung 1 ohne Halogen | Ausgestaltung C-1 |
| 2 | Ausgestaltung 1 ohne Halogen | Ausgestaltung C-2 |
| 3 | Ausgestaltung 1 ohne Halogen | Ausgestaltung C-3 |
| 4 | Ausgestaltung 2 ohne Halogen | Ausgestaltung C-1 |
| 5 | Ausgestaltung 2 ohne Halogen | Ausgestaltung C-2 |
| 6 | Ausgestaltung 2 ohne Halogen | Ausgestaltung C-3 |
| 7 | Ausgestaltung 3 ohne Halogen | Ausgestaltung C-1 |
| 8 | Ausgestaltung 3 ohne Halogen | Ausgestaltung C-2 |
| 9 | Ausgestaltung 3 ohne Halogen | Ausgestaltung C-3 |
| 10 | Ausgestaltung 4 ohne Halogen | Ausgestaltung C-1 |
| 11 | Ausgestaltung 4 ohne Halogen | Ausgestaltung C-2 |
| 12 | Ausgestaltung 4 ohne Halogen | Ausgestaltung C-3 |

Die Verbindungen der Formel (II) lassen sich bevorzugt herstellen durch Kreuzkupplungen, insbesondere durch Negishi Kreuzkupplung der Verbindungen der Formel (IVa) beziehungsweise (IVb) mit den Verbindungen X-Y in Gegenwart eines Katalysators, wie beispielsweise in Angewandte Chemie International Edition 2014, 53, 1430-1434 beschrieben.

Vorzugsweise wird dabei die Verbindung X-Y in einer Gesamtmenge von 0,5 bis 10 Äquivalenten, vorzugsweise von 0,8 bis 5,0 Äquivalenten, weiter bevorzugt von 1,0 bis 2,5 Äquivalenten und besonders bevorzugt von 1,0 bis 2,0 Äquivalenten, bezogen auf die Stoffmenge an Verbindung der Formel (I), eingesetzt.

Schritt b) erfolgt im Falle einer Kreuzkupplung bevorzugt in Gegenwart eines Katalysators. Vorzugsweise ist der Katalysator eine Palladiumverbindung oder eine Nickelverbindung. Besonders bevorzugt ist der Katalysator eine Palladiumverbindung. Ganz besonders bevorzugt handelt es sich um Tetrakis(triphenylphosphine)palladium(0), abgekürzt Pd(PPh₃)₄, der Formel (III).

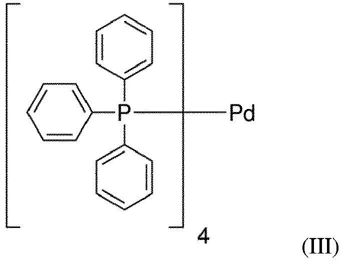

Üblicherweise werden 2,5 bis 25 mol% und vorzugsweise 5 bis 20 mol% Katalysator, insbesondere Tetrakis(triphenylphosphine)palladium(0), eingesetzt.

Schritt b) wird in dieser Variante der Erfindung im Allgemeinen bei einer Temperatur zwischen 0 °C und 120 °C und zunehmend bevorzugt zwischen 10 °C und 100 °C und ganz besonders bevorzugt zwischen 25 °C und 90 °C durchgeführt.

Schritt b) erfolgt in dieser Variante der Erfindung im Allgemeinen in einem Zeitraum von 5 min bis 12 h, bevorzugt von 15 min bis 10 h und besonders bevorzugt von 30 min bis 4 h.

Die Reaktion wird üblicherweise bei Normaldruck durchgeführt, kann aber auch bei erhöhtem bzw. vermindertem Druck durchgeführt werden.

Die erfindungsgemäße Umsetzung der Verbindungen der Formel (I) zu Verbindungen der Formel (IVa) beziehungsweise (IVb) und weiter zu Verbindungen der Formel (II) erfolgt vorzugsweise jeweils in Gegenwart eines organischen Lösungsmittels. Als Lösungsmittel kommen prinzipiell alle organischen Lösungsmittel in Frage, die unter den angewendeten Reaktionsbedingungen inert sind und in denen die umzusetzenden Verbindungen eine ausreichende Löslichkeit aufweisen. Als geeignete Lösungsmittel sind insbesondere zu nennen: Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrolidon (NEP), N-Butyl-2-pyrrolidon (NBP); N,N'-Dimethylpropylenharnstoff (DMPU), Halogenkohlenwasserstoffe und aromatische Kohlenwasserstoffe, insbesondere Chlorkohlenwasserstoffe wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, insbesondere 1,2-Dichlorbenzol, Chlortoluol, Trichlorbenzol; 4-Methoxybenzol, fluorierte Aliphate und Aromaten wie Trichlortrifluorethan, Benzotrifluorid und 4-Chlorbenzotrifluorid.Es können auch Lösungsmittelgemische, vorzugsweise Gemische aus den vorstehend genannten Lösungsmitteln wie Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Dimethylformamid (DMF), eingesetzt werden.

Bevorzugte Lösungsmittel sind THF, N,N-Dimethylformamid (DMF), 1,4-Dioxan, Diglyme, Methyltertbutylether (MTBE), tert-Amyl-methylether (TAME), 2-Methyl-THF, Toluol und 4-Methoxybenzol.

Besonders bevorzugte Lösungsmittel sind THF und N,N-Dimethylformamid (DMF), ganz besonders bevorzugt ist THF.

Das Lösungsmittel kann ferner entgast (sauerstoff-frei) sein.

Vorzugsweise wird für beide Verfahrensschritte a) und b) das gleiche Lösungsmittel verwendet. Alternative erfindungsgemäße Ausgestaltungen, in denen für die Verfahrensschritte a) und b) unterschiedliche Lösungsmittel verwendet werden, sind allerdings ebenfalls möglich, wobei die Lösungsmittel dann ebenfalls vorzugsweise aus den vorstehend genannten Lösungsmitteln ausgewählt sind und die jeweiligen als bevorzugt, besonders bevorzugt und ganz besonders bevorzugt angegebenen Lösungsmittel auf den jeweiligen Verfahrensschritt a) oder b) zu beziehen sind.

Die Isolierung der gewünschten Verbindungen der Formel (II) kann beispielsweise durch wässrige Aufarbeitung in Gegenwart von gesättigten Ammoniumchlorid- oder Natriumthiosulfat-Lösungen und/oder anschließende Chromatographie erfolgen. Solche Verfahren sind dem Fachmann bekannt und schließen auch eine Kristallisation aus einem organischen Lösungsmittel oder Lösungsmittelgemisch ein.

Eine besonders bevorzugte Ausführungsform des erfindungsgemäßen Verfahrens kann anhand des folgenden Schemas (II) erläutert werden:

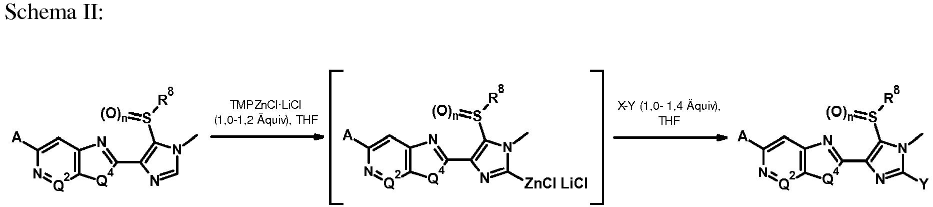

Hierin haben Q², Q⁴, R⁸, n, A, X und Y die vorstehend angegebenen Bedeutungen. Die in Klammern angegebene Verbindung stellt die entsprechende Zwischenstufe der Formel (IVa) dar, welche weiter zum Produkt, einer Verbindung der Formel (II), umgesetzt wird. Beide Umsetzungen finden in THF als Lösungsmittel statt. "Äquiv" bezeichnet die eingesetzte Menge an Äquivalenten TMPZnCl·LiCl bzw. X-Y.

Die Verbindungen der Formel (II) mit Y = Halogen können zudem in einem weiteren Verfahrensschritt umgesetzt werden zu Verbindungen der Formel (II'). 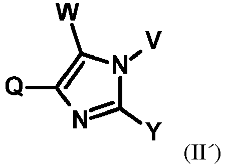 in welcher Q, V, W und Y die oben genannten Bedeutungen und Vorzugsbereiche gemäß einer der Ausgestaltungen 1 bis 20 haben, wobei Y nicht für Halogen steht.

Die Umsetzung der Verbindungen der Formel (II) mit Y = Halogen zu Verbindungen der Formel (II') (Schritt c), erfolgt bevorzugt durch Übergangsmetall-vermittelte Kreuzkupplungen [vgl. Chem. Rev. 1995, 95, 2457-2483; Tetrahedron 2002, 58, 9633-9695; Metal-Catalyzed Cross-Coupling Reactions (Eds.: A. de Meijere, F. Diederich), 2nd ed.,Wiley-VCH, Weinheim, 2004**]** oder durch nukleophile aromatische Substitution (vgl. die in Bioorganic and Medicinal Chemistry Letters 2007, 17, 5825-5830 oder US 4125726 beschriebenen Verfahren).

Beispielsweise können Verbindungen der Formel (II), in denen Y bevorzugt für Brom oder Iod steht, mit geeigneten Boronsäuren [Y-B(OH)₂] oder Boronestern nach bekannten Methoden (vgl. WO 2012/143599, US 2014/094474, US 2014/243316, US 2015/284358 oder Journal of Organic Chemistry 2004, 69, 8829-8835) in Gegenwart geeigneter Katalysatoren aus der Reihe der Übergangsmetallsalze zu Verbindungen der Formel (II') umgesetzt werden. Als Kupplungskatalysatoren kommen dabei beispielsweise bevorzugt Palladiumkatalysatoren wie [1,1'-Bis (diphenylphosphino)ferrocen]dichlorpalladium (II), Bis(triphenylphosphin)palladium(II)dichlorid oder Tetrakis(triphenylphosphin)palladium in Betracht. Als geeignete basische Reaktionshilfsmittel zur Durchführung der Verfahren finden bevorzugt Carbonate des Natriums, Kaliums oder Cäsiums Verwendung. Die benötigten Boronsäurederivate [Y-B(OH)₂] bzw. Boronsäureesterderivate sind teilweise bekannt und/oder kommerziell erhältlich bzw. können nach allgemein bekannten Methoden hergestellt werden (vgl. Boronic Acids (Eds.: D. G. Hall), 2nd ed., Wiley-VCH, Weinheim, 2011**).** Dabei erfolgt die Umsetzung vorzugsweise in einem Gemisch aus Wasser und einem organischen Lösungsmittel, welches ausgewählt ist aus üblichen, bei den vorherrschenden Reaktionsbedingungen inerten Lösungsmitteln. Häufig werden Ether wie beispielsweise Tetrahydrofuran, Dioxan oder 1,2-Dimethoxyethan eingesetzt.

Alternativ können auch Stannanderivate [Y-Sn(*n*-Bu)₄] als Kupplungspartner eingesetzt werden (vgl. US 2013/281433, WO 2004/099177 oder WO 2016/071214). Die benötigten Stannanderivate [Y-Sn(*n*-Bu)₄] sind teilweise bekannt und/oder kommerziell erhältlich bzw. können nach allgemein bekannten Methoden hergestellt werden (vgl. WO 2016/071214 oder WO 2007/148093).

Eine Kupplung der halogenierten Imidazolderivate der Formel (II) mit NH-haltigen Heteroaromaten, wie z. B. Imidazolen oder Pyrazolen, gegebenenfalls. wie oben beschrieben substituiert, zu Verbindungen der Formel (II') kann durch Umsetzung im Basischen (z.B. mit Natriumhydrid in Dimethylformamid, vgl. z.B. WO 2005/058898) erfolgen. Alternativ lässt sich die Umsetzung unter Inertgasatmosphäre durch Katalyse mit Kupfer(I)salzen, beispielsweise Kupfer(I)iodid, in Anwesenheit eines geeigneten Liganden, z. B. (*trans*)-N,N'-Dimethylcyclohexan-1,2-diamin oder *R*-(+)-Prolin, und einer geeigneten Base, z. B. Kaliumcarbonat oder Kaliumphosphat, in einem geeigneten Lösungsmittel, wie z. B. 1,4-Dioxan oder Toluol, durchführen (vgl. z.B. WO 2016/109559).

Ein weiterer Gegenstand der vorliegenden Erfindung sind Verbindungen der Formel (IVa) 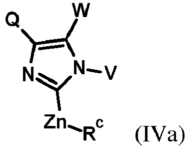 in welcher Q, V, R^{c} und W die oben genannten Bedeutungen und bevorzugten Ausgestaltungen gemäß einer der Ausgestaltungen 1 bis 20 haben.

Die Verbindungen der Formel (IVa) können auch mit Salzen komplexiert vorliegen, wobei es sich bei den Salzen bevorzugt um Alkali- oder Erdalkalihalogenide handelt, vorzugsweise um Lithiumchlorid und/oder Magnesiumchlorid und besonders bevorzugt um Lithiumchlorid.

Unter den Verbindungen der Formel (IVa) sind die folgenden Verbindungen ganz besonders bevorzugt, wobei die jeweilige Verbindung alleine oder als Lithiumchloridkomplex vorliegen kann:

| | | |
|---|---|---|
| IVa-1: | 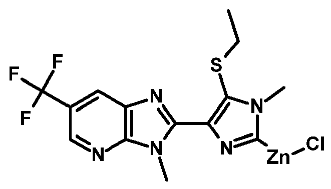 | 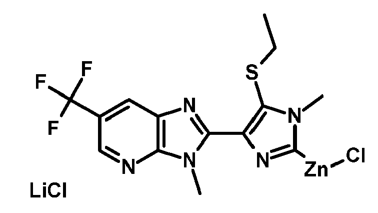 |
| | Chlor{5-(ethylsulfanyl)-1-methyl-4-[3-methyl-6-(trifluormethyl)-3H-imidazo[4,5-b]pyridin-2-yl]-1H-imidazol-2-yl}zink | Chlor{5-(ethylsulfanyl)-1-methyl-4-[3-methyl-6-(trifluormethyl)-3H-imidazo[4,5-b]pyridin-2-yl]-1H-imidazol-2-yl}zink Lithiumchlorid Komplex |
| IVa-2: | 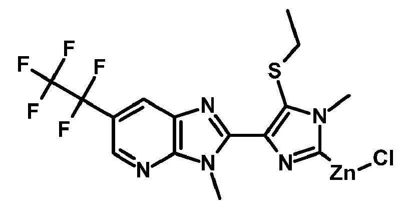 | 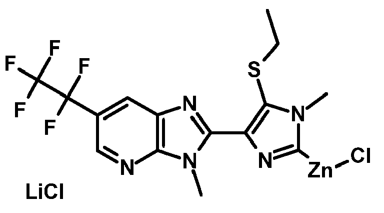 |
| | Chlor{5-(ethylsulfanyl)-1-methyl-4-[3-methyl-6-(pentafluorethyl)-3H-imidazo[4,5-b]pyridin-2-yl]-1H-imidazol-2-yl }zink | Chlor{5-(ethylsulfanyl)-1-methyl-4-[3-methyl-6-(pentafluorethyl)-3H-imidazo[4,5-b]pyridin-2-yl]-1H-imidazol-2-yl}zink Lithiumchlorid Komplex |
| IVa-3: | 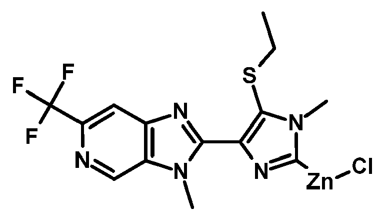 | 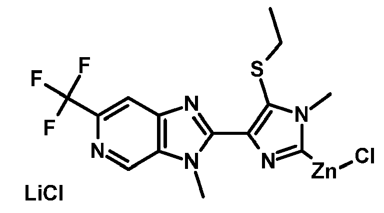 |
| | Chlor{5-(ethylsulfanyl)-1-methyl-4-[3-methyl-6-(trifluormethyl)-3H-imidazo[4,5- | Chlor{5-(ethylsulfanyl)-1-methyl-4-[3-methyl-6-(trifluormethyl)-3H-imidazo[4,5- |
| | c]pyridin-2-yl]-1H-imidazol-2-yl}zink | c]pyridin-2-yl]-1H-imidazol-2-yl}zink Lithiumchlorid Komplex |
| IVa-4: | 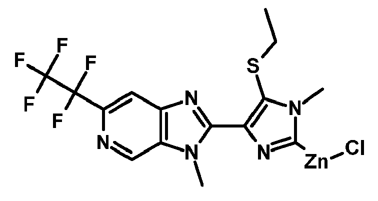 | 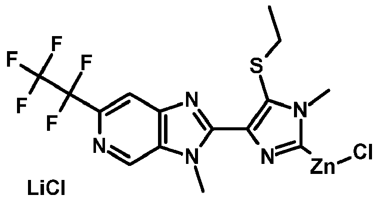 |
| | Chlor{5-(ethylsulfanyl)-1-methyl-4-[3-methyl-6-(pentafluorethyl)-3H-imidazo[4,5-c]pyridin-2-yl]-1H-imidazol-2-yl}zink | Chlor{5-(ethylsulfanyl)-1-methyl-4-[3-methyl-6-(pentafluorethyl)-3H-imidazo[4,5-c]pyridin-2-yl]-1H-imidazol-2-yl}zink Lithiumchlorid Komplex |
| IVa-5: | 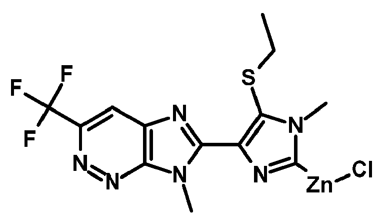 | 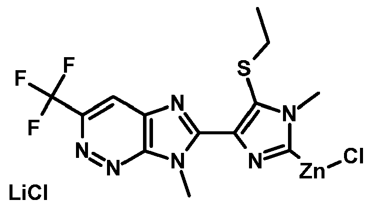 |
| | Chlor{5-(ethylsulfanyl)-1-methyl-4-[7-methyl-3-(trifluormethyl)-7H-imidazo[4,5-c]pyridazin-6-yl]-1H-imidazol-2-yl} zink | Chlor{5-(ethylsulfanyl)-1-methyl-4-[7-methyl-3-(trifluormethyl)-7H-imidazo[4,5-c]pyridazin-6-yl]-1H-imidazol-2-yl}zink Lithiumchlorid Komplex |
| IVa-6: | 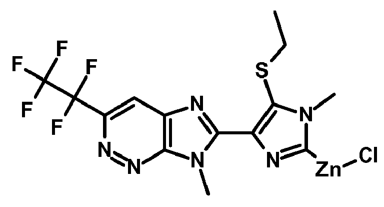 | 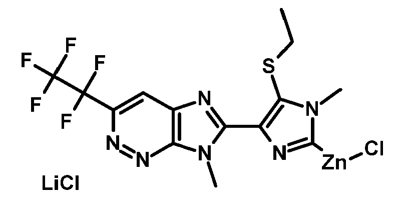 |
| | Chlor{5-(ethylsulfanyl)-1-methyl-4-[7-methyl-3-(pentafluorethyl)-7H-imidazo[4,5-c]pyridazin-6-yl]-1H-imidazol-2-yl}zink | Chlor{5-(ethylsulfanyl)-1-methyl-4-[7-methyl-3-(pentafluorethyl)-7H-imidazo[4,5-c]pyridazin-6-yl]-1H-imidazol-2-yl}zink Lithiumchlorid Komplex |
| IVa-7: | 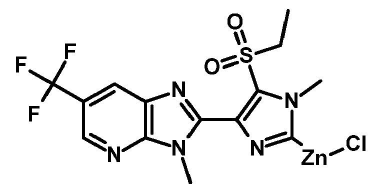 | 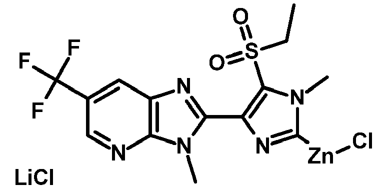 |
| | Chlor{5-(ethylsulfonyl)-1-methyl-4-[3-methyl-6-(trifluormethyl)-3H-imidazo[4,5-b]pyridin-2-yl]-1H-imidazol-2-yl}zink | Chlor{5-(ethylsulfonyl)-1-methyl-4-[3-methyl-6-(trifluormethyl)-3H-imidazo[4,5-b]pyridin-2-yl]-1H-imidazol-2-yl}zink Lithiumchlorid Komplex |
| IVa-8: | 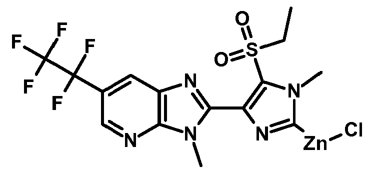 | 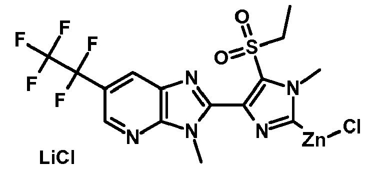 |
| | Chlor{5-(ethylsulfonyl)-1-methyl-4-[3-methyl-6-(pentafluorethyl)-3H-imidazo[4,5-b]pyridin-2-yl]-1H-imidazol-2-yl}zink | Chlor{5-(ethylsulfonyl)-1-methyl-4-[3-b]pyridin-2-yl]-1H-imidazol-2-yl}zink Lithiumchlorid Komplex |
| IVa-9: | 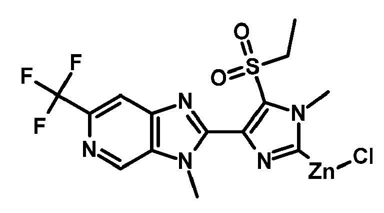 | 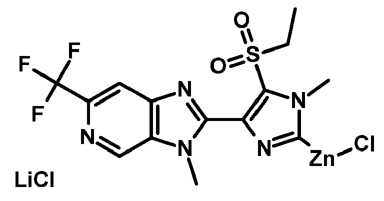 |
| | Chlor{5-(ethylsulfonyl)-1-methyl-4-[3-methyl-6-(trifluormethyl)-3H-imidazo[4,5-c]pyridin-2-yl]-1H-imidazol-2-yl}zink | Chlor{5-(ethylsulfonyl)-1-methyl-4-[3-methyl-6-(trifluormethyl)-3H-imidazo[4,5-c]pyridin-2-yl]-1H-imidazol-2-yl}zink Lithiumchlorid Komplex |
| IVa-10: | 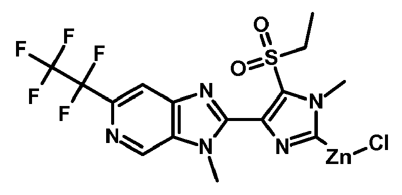 | 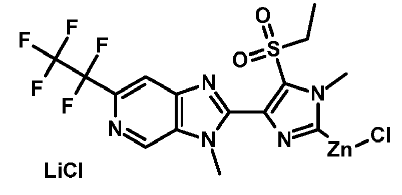 |
| | Chlor{5-(ethylsulfonyl)-1-methyl-4-[3-methyl-6-(pentafluorethyl)-3H-imidazo[4,5-c]pyridin-2-yl]-1H-imidazol-2-yl}zink | Chlor{5-(ethylsulfonyl)-1-methyl-4-[3-methyl-6-(pentafluorethyl)-3H-imidazo[4,5-c]pyridin-2-yl]-1H-imidazol-2-yl}zink Lithiumchlorid Komplex |
| IVa-11: | 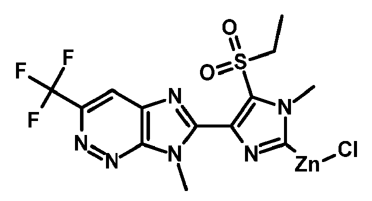 | 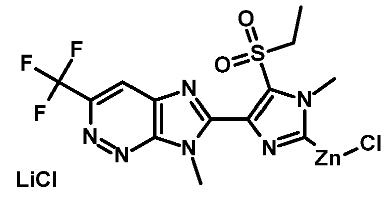 |
| | Chlor{5-(ethylsulfonyl)-1-methyl-4-[7-methyl-3-(trifluormethyl)-7H-imidazo[4,5-c]pyridazin-6-yl]-1H-imidazol-2-yl}zink | Chlor{5-(ethylsulfonyl)-1-methyl-4-[7-methyl-3-(trifluormethyl)-7H-imidazo[4,5-c]pyridazin-6-yl]-1H-imidazol-2-yl}zink Lithiumchlorid Komplex |
| IVa-12: | 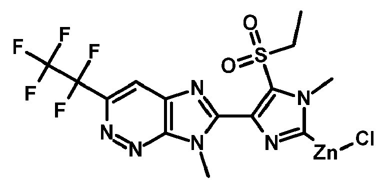 | 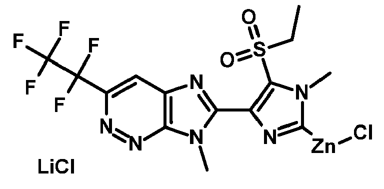 |
| | Chlor{5-(ethylsulfonyl)-1-methyl-4-[7-methyl-3-(pentafluorethyl)-7H-imidazo[4,5-c]pyridazin-6-yl]-1H-imidazol-2-yl}zink | Chlor{5-(ethylsulfonyl)-1-methyl-4-[7-methyl-3-(pentafluorethyl)-7H-imidazo[4,5-c]pyridazin-6-yl]-1H-imidazol-2-yl}zink Lithiumchlorid Komplex |

Ein weiterer Gegenstand der vorliegenden Erfindung sind Verbindungen der Formel (IVb) 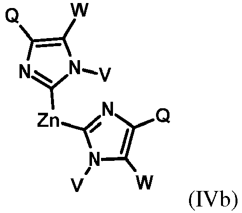 in welcher Q, V und W die oben genannten Bedeutungen und bevorzugten Ausgestaltungen gemäß einer der Ausgestaltungen 1 bis 20 haben.

Die Verbindungen der Formel (IVb) können auch mit Salzen komplexiert vorliegen, wobei es sich bei den Salzen bevorzugt um Alkali- oder Erdalkalihalogenide handelt, vorzugsweise um Lithiumchlorid und/oder Magnesiumchlorid, wie in Struktur (IVb-1) oder (IVb-2) und besonders bevorzugt um Lithiumchlorid (Struktur (IVb-1)).

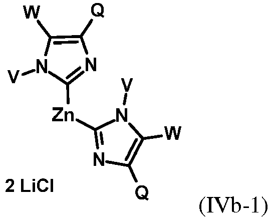

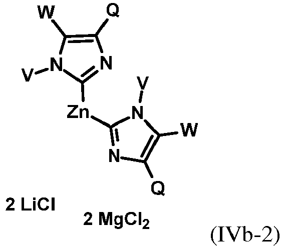

Q, V und W haben in Formel (IVb-1) und (IVb-2) die oben genannten Bedeutungen und bevorzugten Bedeutungen gemäß einer der Ausgestaltungen 1 bis 20.

Die vorliegende Erfindung wird anhand der nachfolgenden Beispiele näher erläutert, wobei die Beispiele nicht in einer die Erfindung einschränkenden Weise zu interpretieren sind.

### Analytische Bestimmungen

Die nachstehend beschriebenen Durchführungen der analytischen Bestimmungen beziehen sich auf alle Angaben im gesamten Dokument, sofern die Durchführung der jeweiligen analytischen Bestimmung an der jeweiligen Textstelle nicht gesondert beschrieben ist.

### Massenspektrometrie

Die Bestimmung von [M+H]⁺ oder M⁻ mittels LC-MS unter sauren chromatographischen Bedingungen wurde mit 1 mL Ameisensäure pro Liter Acetonitril und 0,9 mL Ameisensäure pro Liter Millipore-Wasser als Eluenten durchgeführt. Es wurde die Säule Zorbax Eclipse Plus C18 50 mm ^{∗} 2,1 mm, 1,8 µm verwendet, bei einer Temperatur des Säulenofens von 55 °C.

### Instrumente:

**LC-MS3:** Waters UPLC mit SQD2 Massenspektrometer und SampleManager Probenwechsler. Linearer Gradient 0,0 bis 1,70 Minuten von 10% Acetonitril zu 95% Acetonitril, von 1,70 bis 2,40 Minuten konstant 95% Acetonitril, Fluss 0,85 mL/min.

**LC-MS6 und LC-MS7:** Agilent 1290 LC, Agilent MSD Massenspektrometer, HTS PAL Probenwechsler. Linearer Gradient 0,0 bis 1,80 Minuten von 10% Acetonitril zu 95% Acetonitril, von 1,80 bis 2,50 Minuten konstant 95% Acetonitril, Fluss 1,0 mL/min).

Die Bestimmung von [M+H]⁺ mittels LC-MS unter neutralen chromatographischen Bedingungen wurde mit Acetonitril und Millipore-Wasser mit 79 mg/L Ammoniumcarbonat als Eluenten durchgeführt.

### Instrumente:

**LC-MS4:** Waters IClass Acquity mit QDA Massenspektrometer und FTN Probenwechsler (Säule Waters Acquity 1,7 µm 50 mm ^{∗} 2,1 mm, Säulenofentemperatur 45 °C). Linearer Gradient 0,0 bis 2,10 Minuten von 10% Acetonitril zu 95% Acetonitril, von 2,10 bis 3,00 Minuten konstant 95% Acetonitril, Fluss 0,7 mL/min.

**LC-MS5:** Agilent 1100 LC System mit MSD Massenspektrometer und HTS PAL Probenwechsler (Säule: Zorbax XDB C18 1,8 µm 50 mm ^{∗} 4,6 mm, Säulenofentemperatur 55 °C). Linearer Gradient 0,0 bis 4,25 Minuten von 10% Acetonitril zu 95% Acetonitril, von 4,25 bis 5,80 Minuten konstant 95% Acetonitril, Fluss 2,0 mL/min.

Die Retentionzeit-Indizes wurden in allen Fällen aus einer Kalibrierungsmessung einer homologen Serie von geradkettigen Alkan-2-onen mit 3 bis 16 Kohlenstoffen bestimmt, wobei der Index des ersten Alkanons auf 300, der des letzten auf 1600 gesetzt und zwischen den Werten aufeinanderfolgender Alkanone linear interpoliert wurde.

### logP-Werte

Die Bestimmung der logP-Werte erfolgte gemäß EEC Directive 79/831 Annex V.A8 durch HPLC (High Performance Liquid Chromatography) an einer Phasenumkehrsäule (C18) mit Hilfe folgender Methoden:
Der logP[a] Wert wird durch LC-UV Messung im sauren Bereich bestimmt, mit 0,9 mL/L Ameisensäure in Wasser und 1,0 mL/L Ameisensäure in Acetonitril als Eluenten (linearer Gradient von 10% Acetonitrile bis 95% Acetonitril).

Der logP[n] Wert wird durch LC-UV Messung im neutralen Bereich bestimmt, mit 79 mg/L Ammoniumcarbonat in Wasser und Acetonitril als Eluenten (linearer Gradient von 10% Acetonitril bis 95% Acetonitril).

Die Kalibrierung wurde mit einer homologen Reihe geradkettiger Alkan-2-one (mit 3 bis 16 Kohlenstoffatomen) mit bekannten logP Werten durchgeführt. Die Werte zwischen aufeinanderfolgender Alkanone werden durch lineare Regression bestimmt.

Die Messungen der ¹H-NMR Spektren wurden mit einem Bruker **Avance III 400 MHz** Spektrometer, ausgestattet mit einem 1,7 mm TCI Probenkopf, mit Tetramethylsilan als Standard (0,00 ppm) von Lösungen in den Lösungsmitteln CD₃CN, CDCl₃ oder d₆-DMSO durchgeführt. Alternativ wurde ein **Bruker Avance III 600** MHz Spektrometer ausgestattet mit einem 5 mm CPNMP Probenkopf oder ein Bruker **Avance NEO 600** MHz Spektrometer ausgestattet mit einem 5 mm TCI Probenkopf für die Messungen verwendet. In der Regel wurden die Messungen bei einer Probenkopftemperatur von 298 K durchgeführt. Sofern andere Messtemperaturen verwendet wurden, wird dies gesondert vermerkt.

Die NMR-Daten werden in klassischer Form (δ-Werte, Multiplettaufspaltung, Anzahl der H-Atome) aangegeben.

Das Lösungsmittel, in welchem das NMR-Spektrum aufgenommen wurde ist jeweils angegeben.

### Beispiel 1

### Synthese von 6-[2-Brom-5-(ethylsulfanyl)-1-methyl-1H-imidazol-4-yl]-7-methyl-3-(trifluormethyl)-7H-imidazo[4,5-c]pyridazin:

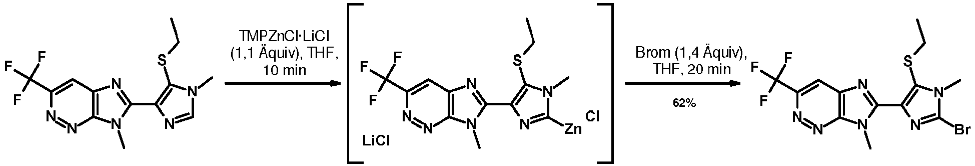

In einem trockenen, mit Argon befüllten Schlenk-Kolben, ausgestattet mit einem Magnetrührfisch und einem Septum, wurde 6-[5-(Ethylsulfanyl)-1-methyl-1H-imidazol-4-yl]-7-methyl-3-(trifluormethyl)-7H-imidazo[4,5-c]pyridazin (1,60 g, 4,67 mmol) in wasserfreiem THF (10 mL) vorgelegt. Zinkchlorid-2,2,6,6-tetramethylpiperidin-1-id Lithiumchlorid Komplex (TMPZnCl·LiCl) (1,31 M in THF, 3,92 mL, 5,14 mmol) wurde tropfenweise zugegeben, und die Mischung wurde 10 min bei 25 °C gerührt. Die Reaktionsmischung wurde auf 0 °C abgekühlt, dann wurde Brom (0,337 mL, 6,54 mmol) zugegeben, und anschließend wurde 20 min bei 25 °C gerührt. Die Reaktionsmischung wurde mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 6-[2-Brom-5-(ethylsulfanyl)-1-methyl-1H-imidazol-4-yl]-7-methyl-3-(trifluormethyl)-7H-imidazo[4,5-c]pyridazin (1,26 g, 62%) als weißen Feststoff ergab.
HPLC-MS: logP[a] = 3,14; logP[n] = 3,01; MH⁺: 421;
¹H-NMR (d₆-DMSO): δ 8,565 (s, 1H), 4,275 (s, 3H), 3,78 (s, 3H), 3,05 (q, 2H), 1,13 (t, 3H).

### Beispiel 2

### Synthese von 6-[2-Brom-5-(ethylsulfanyl)-1-methyl-1H-imidazol-4-yl]-7-methyl-3-(pentafluorethyl)-7H-imidazo[4,5-c]pyridazin:

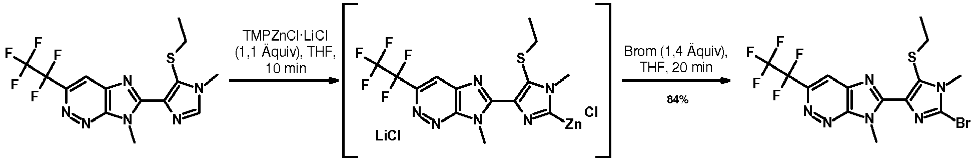

In einem trockenen, mit Argon befüllten Schlenk-Kolben, ausgestattet mit einem Magnetrührfisch und einem Septum, wurde 6-[5-(Ethylsulfanyl)-1-methyl-1H-imidazol-4-yl]-7-methyl-3-(pentafluorethyl)-7H-imidazo[4,5-c]pyridazin (4,00 g, 10,2 mmol) in wasserfreiem THF (20 mL) vorgelegt. Zinkchlorid-2,2,6,6-tetramethylpiperidin-1-id Lithiumchlorid Komplex (TMPZnCl·LiCl) (1,31 M in THF, 8,56 mL, 11,2 mmol) wurde tropfenweise zugegeben, und die Mischung wurde 10 min bei 25 °C gerührt. Die Reaktionsmischung wurde auf 0 °C abgekühlt, dann wurde Brom (0,435 mL, 14,3 mmol) zugegeben, und anschließend wurde 20 min bei 25 °C gerührt. Die Reaktionsmischung wurde mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 6-[2-Brom-5-(ethylsulfanyl)-1-methyl-1H-imidazol-4-yl]-7-methyl-3-(pentafluorethyl)-7H-imidazo[4,5-c]pyridazin (4,36 g, 84%) als weißen Feststoff ergab.
HPLC-MS: logP[a] = 3,80; logP[n] = 3,66; MH⁺: 471;
¹H-NMR (d₆-DMSO): δ 8,59 (s, 1H), 4,28 (s, 3H), 3,79 (s, 3H), 3,06 (q, 2H), 1,12 (t, 3H).

### Beispiel 3

### Synthese von 6-[2-Brom-5-(ethylsulfonyl)-1-methyl-1H-imidazol-4-yl]-7-methyl-3-(pentafluorethyl)-7H-imidazo[4,5-c]pyridazin:

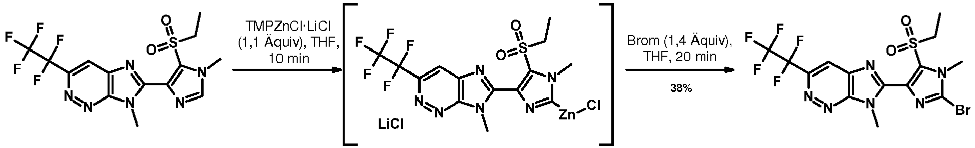

In einem trockenen, mit Argon befüllten Schlenk-Kolben, ausgestattet mit einem Magnetrührfisch und einem Septum, wurde 6-[5-(Ethylsulfonyl)-1-methyl-1H-imidazol-4-yl]-7-methyl-3-(pentafluorethyl)-7H-imidazo[4,5-c]pyridazin (68,0 mg, 0,160 mmol) in wasserfreiem THF (5 mL) vorgelegt. Zinkchlorid-2,2,6,6-tetramethylpiperidin-1-id Lithiumchlorid Komplex (TMPZnCl·LiCl) (1,30 M in THF, 0,136 mL, 0,176 mmol) wurde tropfenweise zugegeben, und die Mischung wurde 10 min bei 25 °C gerührt. Die Reaktionsmischung wurde auf 0 °C abgekühlt, dann wurde Brom (11 µL, 0,224 mmol) zugegeben, und anschließend wurde 20 min bei 25 °C gerührt. Die Reaktionsmischung wurde mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 6-[2-Brom-5-(ethylsulfonyl)-1-methyl-1H-imidazol-4-yl]-7-methyl-3-(pentafluorethyl)-7H-imidazo[4,5-c]pyridazin (40,0 mg, 50%) als weißen Feststoff ergab.
HPLC-MS: logP[a] = 2,80; logP[n] = 2,76; MH⁺: 503;
¹H-NMR (d₆-DMSO): δ 8,74 (s, 1H), 4,06 (s, 3H), 3,94 (s, 3H), 3,78 (q, 2H), 1,29 (t, 3H).

### Beispiel 4

### Synthese von 2-[2-Brom-5-(ethylsulfanyl)-1-methyl-1H-imidazol-4-yl]-3-methyl-6-(pentafluorethyl)-3H-imidazo[4,5-c]pyridin:

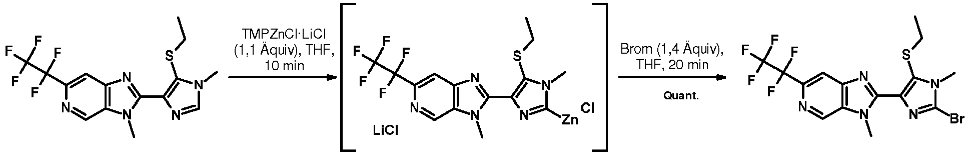

In einem trockenen, mit Argon befüllten Schlenk-Kolben, ausgestattet mit einem Magnetrührfisch und einem Septum, wurde 2-[5-(Ethylsulfanyl)-1-methyl-1H-imidazol-4-yl]-3-methyl-6-(pentafluorethyl)-3H-imidazo[4,5-c]pyridin (86,5 mg, 0,221 mmol) in wasserfreiem THF (5 mL) vorgelegt. Zinkchlorid-2,2,6,6-tetramethylpiperidin-1-id Lithiumchlorid Komplex (TMPZnCl·LiCl) (1,30 M in THF, 0,187 mL, 0,243 mmol) wurde tropfenweise zugegeben, und die Mischung wurde 10 min bei 25 °C gerührt. Die Reaktionsmischung wurde auf 0 °C abgekühlt, dann wurde Brom (16 µL, 0,309 mmol) zugegeben, und anschließend wurde 20 min bei 25 °C gerührt. Die Reaktionsmischung wurde mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 2-[2-Brom-5-(ethylsulfanyl)-1-methyl-1H-imidazol-4-yl]-3-methyl-6-(pentafluorethyl)-3H-imidazo[4,5-c]pyridin (128 mg, Quant.) als weißen Feststoff ergab.
HPLC-MS: logP[a] = 3,63; logP[n] = 3,51; MH⁺: 470;
¹H-NMR (d₆-DMSO): δ 9,17 (s, 1H), 8,21 (s, 1H), 4,15 (s, 3H), 3,76 (s, 3H), 3,01 (q, 2H), 1,11 (t, 3H).

### Beispiel 5

### Synthese von 6-[2-(6-Chlorpyridazin-3-yl)-5-(ethylsulfanyl)-1-methyl-1H-imidazol-4-yl]-7-methyl-3-(pentafluorethyl)-7H-imidazo[4,5-c]pyridazin:

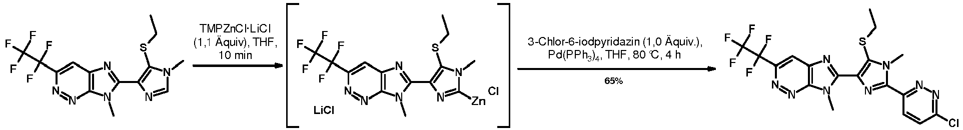

In einem trockenen, mit Argon befüllten Schlenk-Kolben, ausgestattet mit einem Magnetrührfisch und einem Septum, wurde 6-[5-(Ethylsulfonyl)-1-methyl-1H-imidazol-4-yl]-7-methyl-3-(pentafluorethyl)-7H-imidazo[4,5-c]pyridazin (100 mg, 0,255 mmol) in wasserfreiem THF (5 mL) vorgelegt. Zinkchlorid-2,2,6,6-tetramethylpiperidin-1-id Lithiumchlorid Komplex (TMPZnCl·LiCl) (1,31 M in THF, 0,214 mL, 0,280 mmol) wurde tropfenweise zugegeben, und die Mischung wurde 10 min bei 25 °C gerührt. Eine Lösung von 3-Chlor-6-iodpyridazin (61,3 mg, 0, 255 mmol) in wasserfreiem THF (10 mL) sowie Tetrakis(triphenylphosphin)palladium(0) (29,5 mg, 0,025 mmol) wurden zugegeben, und anschließend wurde die Reaktionsmischung 4 Stunden bei 80 °C gerührt. Die Reaktionsmischung wurde auf 25 °C abgekühlt, mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 6-[2-(6-Chlorpyridazin-3-yl)-5-(ethylsulfanyl)-1-methyl-1H-imidazol-4-yl]-7-methyl-3-(pentafluorethyl)-7H-imidazo[4,5-c]pyridazin (83,3 mg, 65%) als weißen Feststoff ergab.
HPLC-MS: logP[a] = 4,13; logP[n] = 4,01; MH⁺: 505;
¹H-NMR (d₆-DMSO): δ 8,63 (s, 1H), 8,54 (d, 1H), 8,14 (d, 1H), 4,41 (s, 3H), 4,26 (s, 3H), 3,14 (q, 2H), 1,18 (t, 3H).

### Beispiel 6

### Synthese von 6-{5-(Ethylsulfanyl)-1-methyl-4-[7-methyl-3-(pentafluorethyl)-7H-imidazo[4,5-c]pyridazin-6-yl]-1H-imidazol-2-yl}nicotinonitril:

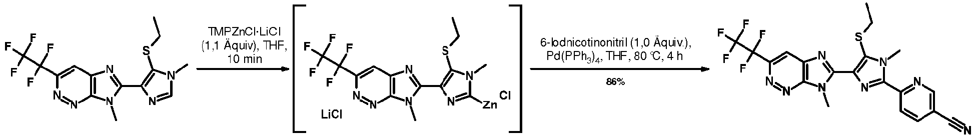

In einem trockenen, mit Argon befüllten Schlenk-Kolben, ausgestattet mit einem Magnetrührfisch und einem Septum, wurde 6-[5-(Ethylsulfonyl)-1-methyl-1H-imidazol-4-yl]-7-methyl-3-(pentafluorethyl)-7H-imidazo[4,5-c]pyridazin (100 mg, 0,255 mmol) in wasserfreiem THF (5 mL) vorgelegt. Zinkchlorid-2,2,6,6-tetramethylpiperidin-1-id Lithiumchlorid Komplex (TMPZnCl·LiCl) (1,31 M in THF, 0,214 mL, 0,280 mmol) wurde tropfenweise zugegeben, und die Mischung wurde 10 min bei 25 °C gerührt. Eine Lösung von 6-Iodnicotinonitril (58,6 mg, 0, 255 mmol) in wasserfreiem THF (10 mL) sowie Tetrakis(triphenylphosphin)palladium(0) (29,5 mg, 0,025 mmol) wurden zugegeben, und anschließend wurde die Reaktionsmischung 4 Stunden bei 80 °C gerührt. Die Reaktionsmischung wurde auf 25 °C abgekühlt, mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 6-{5-(Ethylsulfanyl)-1-methyl-4-[7-methyl-3-(pentafluorethyl)-7H-imidazo[4,5-c]pyridazin-6-yl]-1H-imidazol-2-yl}nicotinonitril (109 mg, 86%) als weißen Feststoff ergab.
HPLC-MS: logP[a] = 4,32; logP[n] = 4,20; MH⁺: 495;
¹H-NMR (d₆-DMSO): δ 9,19 (m, 1H), 8,62 (s, 1H), 8,51 (m, 1H), 8,46 (m, 1H), 4,42 (s, 3H), 4,265 (s, 3H), 3,13 (q, 2H), 1,16 (t, 3H).

## Patentansprüche

1. Verfahren zur Herstellung von Verbindungen der Formel (II) 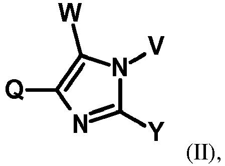 in welcher
Q für ein Strukturelement 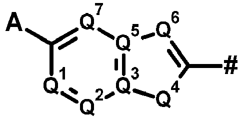
steht, wobei das Zeichen # die Bindung zum Rest des Moleküls anzeigt und
Q¹ für N oder CR⁶ steht,
Q² für N oder CR⁶ steht,
Q³ für N oder C steht,
Q⁴ für O, S, N, CR⁶ oder NR⁷ steht,
Q⁵ für N oder C steht,
Q⁶ für N oder CH steht und
Q⁷ für N oder CH steht,
wobei maximal fünf der Variablen Q¹, Q², Q³, Q⁴, Q⁵, Q⁶ und Q⁷ gleichzeitig für Stickstoff stehen und Q³ und Q⁵ nicht gleichzeitig für N stehen und
R⁶ für Wasserstoff, (C₁-C₄)Alkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht und
R⁷ für (C₁-C₄)Alkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl,Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
A für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl,(C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonyl steht, oder
A für -O-CF₂-O- steht und gemeinsam mit Q¹ und dem Kohlenstoffatom, an das es gebunden ist, einen fünfgliedrigen Ring bildet, wobei Q¹ für Kohlenstoff steht,
W für Halogen oder S(O)ₙR⁸ steht, wobei
R⁸ für (C₁-C₆)Alkyl, (C₁-C₆)Cyanoalkyl, (C₁-C₆)Alkoxy-(C₁-C₆)alkyl, (C₁-C₆)Halogenalkyl, (C₂-C₆)Alkenyl, (C₂-C₆)Halogenalkenyl, (C₂-C₆)Alkinyl, (C₂-C₆)Halogenalkinyl oder (C₃-C₈)Cycloalkyl steht und
n für 0, 1 oder 2 steht,
V für (C₁-C₆)-Alkyl, (C₁-C₆)Halogenalkyl, (C₁-C₆)Cyanoalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₈)Cycloalkyl-(C₃-C₈)Cycloalkyl, (C₁-C₆)Alkyl-(C₃-C₈)Cycloalkyl, Halogen(C₃-C₈)cycloalkyl, Cyano(C₃-C₈)cycloalkyl, (C₁-C₆)Alkylthio-(C₁-C₆)alkyl, (C₁-C₆)Halogenaₗkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl oder (C₁-C₆)Halogenalkoxycarbonyl-(C₁-C₆)alkyl steht und
Y für Halogen, Cyano, Nitro, (C₁-C₄)Alkyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, SCN, (C₁-C₄)Alkylcarbonyl, (C₁-C₄)Halogenalkylcarbonyl, (C₁-C₄)Alkoxycarbonyl, (C₁-C₄)Halogenalkoxycarbonyl, Aminocarbonyl, (C₁-C₄)Alkylaminocarbonyl, Di-(C₁-C₄)alkylaminocarbonyl, (C₁-C₄)Halogenalkylaminocarbonyl, (C₃-C₆)Cycloalkylaminocarbonyl, Aminothiocarbonyl, (C₁-C₄)Alkylaminothiocarbonyl, Di-(C₁-C₄)alkylaminothiocarbonyl, (C₁-C₄)Halogenalkylaminothiocarbonyl, (C₃-C₆)Cycloalkylaminothiocarbonyl, Amino, (C₁-C₄)Alkylamino, (C₁-C₄)Halogenalkylamino, Di-(C₁-C₄)Alkylamino,(C₃-C₆)Cycloalkylamino, (C₁-C₄)Alkylsulfonylamino, (C₁-C₄)Alkylcarbonylamino, (C₁-C₄)Halogenalkylcarbonylamino, (C₁-C₄)Alkylcarbonyl-(C₁-C₄)alkyl-amino, (C₁-C₄)Halogenalkylcarbonyl-(C₁-C₄)alkyl-amino, (C₃-C₆)Cycloalkylcarbonylamino, (C₃-C₆)Cycloalkylcarbonyl-(C₁-C₄)alkyl-amino, (C₁-C₄)Alkylthiocarbonylamino, (C₁-C₄)Halogenalkylthiocarbonylamino, (C₁-C₄)Alkylthiocarbonyl-(C₁-C₄)alkyl-amino, (C₁-C₄)Halogenalkylthiocarbonyl-(C₁-C₄)alkyl-amino, (C₃-C₆)Cycloalkylthiocarbonylamino, (C₃-C₆)Cycloalkylthiocarbonyl-(C₁-C₄)alkyl-amino, (C₂-C₄)Alkenyl, (C₂-C₄)Halogenalkenyl, (C₂-C₄)Cyanoalkenyl, (C₃-C₆)Cycloalkyl-(C₂)alkenyl, (C₂-C₄)Alkinyl oder (C₂-C₄)Halogenalkinyl steht,
oder für jeweils gegebenenfalls einfach oder mehrfach, gleich oder verschieden substituiertes (C₃-C₆)Cycloalkyl oder (C₅-C₆)Cycloalkenyl steht, wobei als Substituenten jeweils in Frage kommen: (C₃-C₆)Cycloalkyl, (C₁-C₄)Alkyl, (C₁-C₄)Halogenalkyl, (C₁-C₄)Alkoxy, (C₁-C₄)Halogenalkoxy, Aminocarbonyl, Aminothiocarbonyl, Halogen oder Cyano,
oder für jeweils gegebenenfalls einfach oder mehrfach, gleich oder verschieden substituiertes Aryl oder Hetaryl steht, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, SF₅, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl,(C₁-C₄)Halogenalkyl-(C₃-C₆)cycloalkyl, Halogen-(C₃-C₆)cycloalkyl, Cyano-(C₃-C₆)cycloalkyl, (C₁-C₄)Alkyl, (C₁-C₄)Halogenalkyl, (C₁-C₄)Cyanoalkyl, (C₁-C₄)Hydroxyalkyl, Hydroxycarbonyl-(C₁-C₄)-alkoxy, (C₁-C₄)Alkoxycarbonyl-(C-C₄)alkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₃-C₆)Cycloalkyl-(C₂)alkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxycarbonyl-(C₁-C₄)alkoxy, (C₁-C₄)Alkoxy-(C₁-C₄)alkoxy, (C₁-C₄)Alkoxyimino, (C₁-C₄)Halogenalkoxyimino, (C₁-C₄)Alkylthio, (C₁-C₄)Halogenalkylthio, (C₁-C₄)Alkoxy-(C₁-C₄)alkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkoxy-(C₁-C₄)alkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkoxy-(C₁-C₄)alkylsulfonyl, (C₁-C₄)Alkylsulfonyl-(C₁-C₄)alkyl, (C₁-C₄)Alkylsulfonyloxy, (C₁-C₄)Halogenalkylsulfonyloxy, (C₁-C₄)Alkylcarbonyl, (C₁-C₄)Halogenalkylcarbonyl, (C₁-C₄)Alkylcarbonyloxy, (C₁-C₄)Alkoxycarbonyl, (C₁-C₄)Halogenalkoxycarbonyl, Aminocarbonyl, (C₁-C₄)Alkylaminocarbonyl, (C₁-C₄)Halogenalkylaminocarbonyl, Di-(C₁-C₄)alkyl-aminocarbonyl, (C₂-C₄)Alkenylaminocarbonyl, Di-(C₂-C₄)-alkenylaminocarbonyl, (C₃-C₆)Cycloalkylaminocarbonyl, (C₁-C₄)Alkylsulfonylamino, (C₁-C₄)Alkylamino, Di-(C₁-C₄)Alkylamino, (C₁-C₄)Halogenalkylamino, (C₃-C₆)Cycloalkylamino, Aminosulfonyl, (C₁-C₄)Alkylaminosulfonyl, Di-(C₁-C₄)alkylaminosulfonyl, (C₁-C₄)Alkylsulfoximino, Aminothiocarbonyl, (C₁-C₄)Alkylaminothiocarbonyl, Di-(C₁-C₄)alkylaminothiocarbonyl, (C₁-C₄)Halogenalkylaminothiocarbonyl, (C₃-C₆)Cycloalkylaminothiocarbonyl, (C₁-C₄)Alkylcarbonylamino, (C₁-C₄)Halogenalkylcarbonylamino, (C₁-C₄)Alkylcarbonyl-(C₁-C₄)alkylamino, (C₁-C₄)Halogenalkylcarbonyl-(C₁-C₄)alkyl-amino, (C₃-C₆)Cycloalkylcarbonylamino, (C₃-C₆)Cycloalkylcarbonyl-(C₁-C₄)alkyl-amino, (C₁-C₄)Alkylthiocarbonylamino, (C₁-C₄)Halogenalkylthiocarbonylamino, (C₁-C₄)Alkylthiocarbonyl-(C₁-C₄)alkyl-amino, (C₁-C₄)Halogenalkylthiocarbonyl-(C₁-C₄)alkyl-amino, (C₃-C₆)Cycloalkylthiocarbonylamino, (C₃-C₆)Cycloalkylthiocarbonyl-(C₁-C₄)alkyl-amino, Hetaryl, Oxo-Hetaryl, Halogen-Hetaryl, Halogen-Oxo-Hetaryl, Cyano-Hetaryl, Cyano-Oxo-Hetaryl, (C₁-C₄)Halogenalkyl-Hetaryl oder (C₁-C₄)Halogenalkyl-Oxo- Hetaryl,
**dadurch gekennzeichnet, dass** in einem ersten Verfahrensschritt a) eine Verbindung der Formel (I) 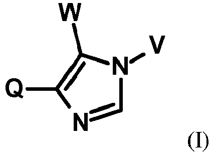
in welcher Q, V und W jeweils die oben genannten Bedeutungen haben,
mit einer Zink-metallorganischen Base der Struktur (NR^{a}R^{b})-Zn-R^{c} oder (NR^{a}R^{b})₂-Zn, in welcher
R^{c} für Halogen oder -O-Pivaloyl steht und
R^{a} und R^{b} gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R^{d}-Radikale substituiert sein kann und R^{d} ausgewählt ist aus der Gruppe bestehend aus Methyl, Ethyl, n-Propyl und i-Propyl,
zu einer Verbindung der Formel (IVa) oder der Formel (IVb) umgesetzt wird, 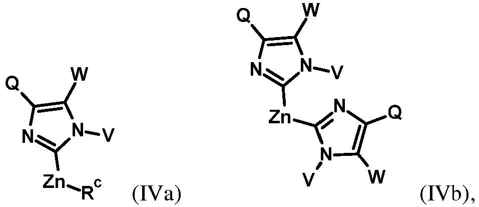
in welcher Q, V, W und R^{c} jeweils die oben genannten Bedeutungen haben,
und diese Verbindung der Formel (IVa) oder (IVb) in einem zweiten Verfahrensschritt b) mit einer Verbindung der Struktur Y-X, in welcher X für Halogen steht und Y die oben genannte Bedeutung hat, zur Verbindung der Formel (II) umgesetzt wird.

2. Verfahren gemäß Anspruch 1, **dadurch gekennzeichnet, dass**
Q für ein Strukturelement aus der Reihe O1 bis Q14 steht, 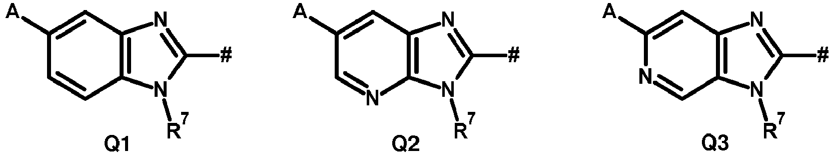 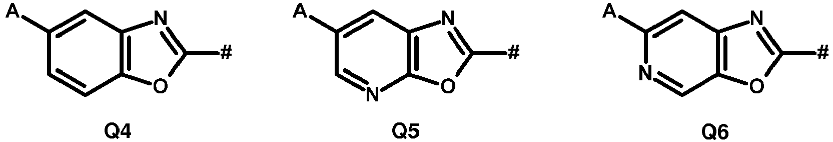 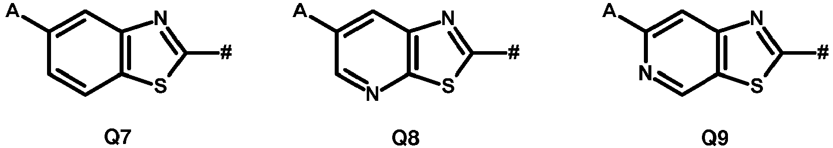 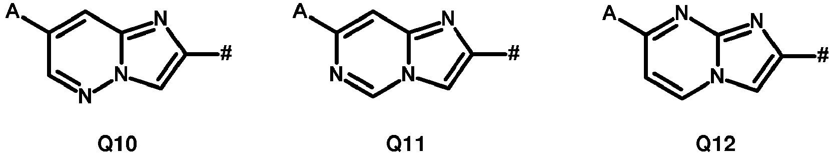 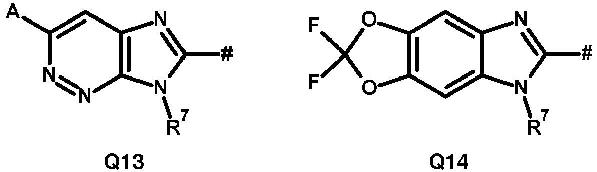
wobei
R⁷ für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht und
A für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl steht,
W für Halogen oder S(O)ₙR⁸ steht, wobei
R⁸ für (C₁-C₆)Alkyl, (C₁-C₆)Cyanoalkyl, (C₁-C₆)Alkoxy-(C₁-C₆)alkyl, (C₁-C₆)Halogenalkyl, (C₂-C₆)Alkenyl, (C₂-C₆)Halogenalkenyl, (C₂-C₆)Alkinyl, (C₂-C₆)Halogenalkinyl oder (C₃-C₈)Cycloalkyl steht und
n für 0, 1 oder 2 steht,
R^{c} für Halogen, insbesondere Chlor, Brom oder Iod steht,
V für (C₁-C₆)-Alkyl, C₁-C₆)Cyanoalkyl, (C₁-C₆)Alkoxy-(C₁-C₆)alkyl, (C₁-C₆)Halogenalkyl, (C₂-C₆)Alkenyl, (C₂-C₆)Halogenalkenyl, (C₂-C₆)Alkinyl, (C₂-C₆)Halogenalkinyl oder (C₃-C₈)Cycloalkyl steht und
Y für Halogen, Cyano, (C₁-C₄)Alkyl, (C₁-C₄)Halogenalkyl, (C₁-C₄)Alkoxy, (C₁-C₄)Halogenalkoxy, Aminocarbonyl, (C₁-C₄)Alkylaminocarbonyl, Di-(C₁-C₄)alkylaminocarbonyl, (C₁-C₄)Halogenalkylaminocarbonyl, (C₃-C₆)Cycloalkylaminocarbonyl, Amino, (C₁-C₄)Alkylamino, (C₁-C₄)Halogenalkylamino, Di-(C₁-C₄)Alkylamino, (C₃-C6)Cycloalkylamino, (C₁-C₄)Alkylsulfonylamino, (C₁-C₄)Alkylcarbonylamino, (C₁-C₄)Halogenalkylcarbonylamino, (C₁-C₄)Alkylcarbonyl-(C₁-C₂)alkyl-amino, (C₁-C₄)Halogenalkylcarbonyl-(C₁-C₂)alkyl-amino, (C₃-C₆)Cycloalkylcarbonylamino, (C₃-C₆)Cycloalkylcarbonyl-(C₁-C₂)alkyl-amino, (C₂-C₄)Alkenyl, (C₂-C₄)Halogenalkenyl, (C₂-C₄)Cyanoalkenyl oder (C₃-C₆)Cycloalkyl-(C₂)alkenyl,
oder für jeweils gegebenenfalls einfach oder mehrfach, gleich oder verschieden substituiertes (C₃-C₆)Cycloalkyl oder (C₅-C₆)Cycloalkenyl steht, wobei als Substituenten jeweils in Frage kommen: (C₃-C₆)Cycloalkyl, (C₁-C₄)Alkyl, (C₁-C₄)Halogenalkyl, (C₁-C₄)Alkoxy, (C₁-C₄)Halogenalkoxy, Halogen oder Cyano,
oder für jeweils gegebenenfalls einfach oder mehrfach, gleich oder verschieden substituiertes Phenyl, Pyridyl, Pyrimidyl, Pyridazinyl, Thiophenyl, Furanyl, Pyrazolyl, Pyrrolyl, Thiazolyl, Oxazolyl oder Imidazolyl steht, wobei als Substituenten jeweils in Frage kommen: Cyano, Halogen, Nitro, Acetyl, Hydroxy, Amino, SF₅-, (C₃-C₆)Cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₂)alkyl, (C₂-C₄)Alkenyl, (C₂-C₄)Halogenalkenyl, (C₂-C₄)Cyanoalkenyl, (C₃-C₆)Cycloalkyl-(C₂)alkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₂)alkoxy, (C₁-C₄)Alkoxyimino, (C₁-C₄)Halogenalkoxyimino, (C₁-C₄)Alkylthio, (C₁-C₄)Halogenalkylthio, (C₁-C₄)Alkylthio-(C₁-C₂)alkyl, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Halogenalkylsulfonyloxy, (C₁-C₄)Alkylcarbonyl, (C₁-C₄)Halogenalkylcarbonyl, Aminocarbonyl, (C₁-C₄)Alkylaminocarbonyl, (C₁-C₄)Halogenalkylaminocarbonyl, Di-(C₁-C₄)alkyl-aminocarbonyl, (C₃-C₆)Cycloalkylaminocarbonyl, Aminothiocarbonyl, (C₁-C₄)Alkylaminothiocarbonyl, Di-(C₁-C₄)alkylaminothiocarbonyl, (C₁-C₄)Halogenalkylaminothiocarbonyl, (C₃-C₆)Cycloalkylaminothiocarbonyl, (C₁-C₄)Alkylsulfonylamino, (C₁-C₄)Alkylamino, Di-(C₁-C₄)Alkylamino, (C₁-C₄)Halogenalkylamino, (C₃-C₆)Cycloalkylamino, Aminosulfonyl, (C₁-C₄)Alkylaminosulfonyl, Di-(C₁-C₄)alkyl-aminosulfonyl, (C₁-C₄)Alkylcarbonylamino, (C₁-C₄)Halogenalkylcarbonylamino, (C₁-C₄)Alkylcarbonyl-(C₁-C₂)alkyl-amino, (C₁-C₂)Halogenalkylcarbonyl-(C₁-C₂)alkyl-amino, (C₃-C₆)Cycloalkylcarbonylamino, (C₃-C₆)Cycloalkylcarbonyl-(C₁-C₂)alkyl-amino, (C₁-C₄)Alkylthiocarbonylamino, (C₁-C₄)Halogenalkylthiocarbonylamino, (C₁-C₄)Alkylthiocarbonyl-(C₁-C₂)alkyl-amino, (C₁-C₄)Halogenalkylthiocarbonyl-(C₁-C₂)alkyl-amino, (C₃-C₆)Cycloalkylthiocarbonylamino oder (C₃-C₆)Cycloalkylthiocarbonyl-(C₁-C₂)alkyl-amino.

3. Verfahren gemäß Anspruch 1, **dadurch gekennzeichnet, dass**
Q für ein Strukturelement aus der Reihe Q2, Q3, Q4, Q10, Q11, Q13 oder Q14 steht, wobei
R⁷ für (C₁-C₄)Alkyl oder (C₁-C₄)Alkoxy-(C₁-C₄)alkyl steht und
A für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl steht,
W für Halogen oder S(O)ₙR⁸ steht, wobei
R⁸ für Methyl, Ethyl, n-Propyl oder iso-Propyl steht und
n für 0,1 oder 2 steht,
R^{c} für Chlor steht,
V für Methyl, Ethyl, n-Propyl oder iso-Propyl steht und
Y für Brom, Iod, Cyano, Ethenyl, Cyclopropylethenyl, i-Propenyl, Cyclopropylethinyl, Methyl, Ethyl, Isopropyl, Cyclopropylethyl, Methoxycarbonyl, Trifluorethylaminocarbonyl, Aminocarbonyl, Methylaminocarbonyl, Dimethylaminocarbonyl, Ethylaminocarbonyl, Aminothiocarbonyl, Methylaminothiocarbonyl, Dimethylaminothiocarbonyl,
oder für jeweils gegebenenfalls einfach oder zweifach, gleich oder verschieden substituiertes Cyclopropyl, Cyclobutyl, Cyclopentyl, Cyclohexyl, Cyclopentenyl oder Cyclohexenyl steht, wobei als Substituenten jeweils in Frage kommen: Methyl, Ethyl, n-Propyl, i-Propyl, cyclo-Propyl, Difluormethyl, Trifluormethyl, Cyano, Fluor oder Chlor,
oder für jeweils gegebenenfalls einfach, zweifach oder dreifach, gleich oder verschieden substituiertes Phenyl, Pyridin-2-yl, Pyridin-3-yl, Pyridin-4-yl, Pyrimidin-5-yl, Pyridazin-3-yl, Pyridazin-4-yl, Thien-2-yl, Thien-3-yl, 1,3-Thiazol-5-yl, 1H-Imidazol-1-yl, 1H-Imidazol-2-yl, 1H-Imidazol-5-yl, 1H-Pyrazol-1-yl, 1H-Pyrazol-3-yl, 1H-Pyrazol-4-yl, 1H-Pyrazol-5-yl, 1H-Pyrrol-1-yl, 1H-Pyrrol-2-yl, 1H-Pyrrol-3-yl oder 1-Cyclohexenyl steht, wobei als Substituenten jeweils in Frage kommen: Cyano, Fluor, Chlor, Methyl, Cyclopropyl, Cyanomethyl, Cyanoisopropyl, Cyanocylopropyl, Trifluormethyl, Trifluorethyl oder Aminocarbonyl.

4. Verfahren gemäß Anspruch 1, **dadurch gekennzeichnet, dass**
Q für das Strukturelement Q3 oder Q13 steht, wobei
R⁷ für Methyl oder Ethyl, insbesondere für Methyl steht und
A für Trifluormethyl oder Pentafluorethyl steht,
W für S(O)ₙR⁸ steht, wobei
R⁸ für Ethyl steht und
n für 0 oder 2 steht,
R^{c} für Chlor steht,
V für Methyl oder Ethyl, insbesondere für Methyl steht und
Y für Brom, 5-Cyanpyridin-2-yl oder 6-Chlorpyridazin-3-yl steht.

5. Verfahren gemäß einem der Ansprüche 1 bis 4, **dadurch gekennzeichnet, dass** es sich bei der Zink-metallorganischen Base um eine Verbindung der Formel (VI) handelt
(VI) (TMP)ₓ ZnCl₂₋ₓ ,
worin x für die Zahl 1 oder 2 steht.

6. Verfahren gemäß einem der Ansprüche 1 bis 5, **dadurch gekennzeichnet, dass** die Zinkmetallorganische Base in Verbindung mit einem Alkali- oder Erdalkalihalogenid, vorzugsweise Lithiumchlorid und/oder Magnesiumchlorid, vorliegt.

7. Verfahren gemäß einem der Ansprüche 1 bis 6, **dadurch gekennzeichnet, dass** Verfahrensschritt a) bei einer Temperatur zwischen 0 °C und 110 °C durchgeführt wird.

8. Verfahren gemäß einem der Ansprüche 1 bis 7, **dadurch gekennzeichnet, dass** X für Brom oder Iod stehen.

9. Verfahren gemäß einem der Ansprüche 1 bis 8, **dadurch gekennzeichnet, dass** Y für Halogen stehen.

10. Verfahren gemäß Anspruch 9, **dadurch gekennzeichnet, dass** es sich bei der Verbindung X-Y um ein elementares Halogen, insbesondere um F₂, Cl₂, Br₂ oder I₂, handelt.

11. Verfahren gemäß einem der Ansprüche 1 bis 8, **dadurch gekennzeichnet, dass** Y definiert ist gemäß einem der Ansprüche 1 bis 4, aber nicht für Halogen steht.

12. Verfahren gemäß einem der Ansprüche 9 oder 10, **dadurch gekennzeichnet, dass** Verfahrensschritt b) bei einer Temperatur zwischen 0 °C und 80 °C durchgeführt wird.

13. Verfahren gemäß Anspruch 11, **dadurch gekennzeichnet, dass** Verfahrensschritt b) bei einer Temperatur zwischen 0 °C und 120 °C durchgeführt wird.

14. Verfahren gemäß einem der Ansprüche 1 bis 13, **dadurch gekennzeichnet, dass** Verfahrensschritt a) und Verfahrensschritt b) in Gegenwart eines organischen Lösungsmittel durchgeführt werden, wobei das Lösungsmittel ausgewählt ist aus THF oder N,N-Dimethylformamid (DMF).

15. Verbindungen der Formel (IVa) 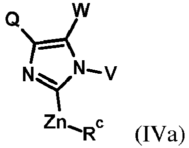 in welcher Q, V, R^{c} und W die Bedeutungen gemäß einem der Ansprüche 1 bis 4 haben.

16. Verbindungen der Formel (IVb) 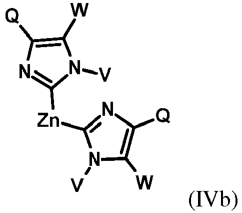 in welcher Q, V und W die Bedeutungen gemäß einem der Ansprüche 1 bis 4 haben.

17. Verbindungen der Formel (IVa) gemäß Anspruch 15 oder (IVb) gemäß Anspruch 16, **dadurch gekennzeichnet, dass** diese mit Salzen komplexiert vorliegen, wobei es sich bei den Salzen um Alkali- oder Erdalkalihalogenide handelt, vorzugsweise um Lithiumchlorid und/oder Magnesiumchlorid.

## Claims

1. Process for preparing compounds of the formula (II) 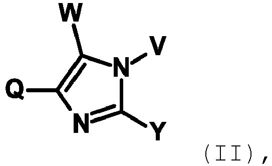 in which
Q represents a structural element 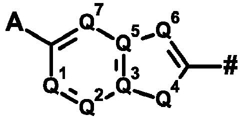
where the symbol # indicates the bond to the rest of the molecule and
Q¹ represents N or CR⁶,
Q² represents N or CR⁶,
Q³ represents N or C,
Q⁴ represents 0, S, N, CR⁶ or NR⁷,
Q⁵ represents N or C,
Q⁶ represents N or CH and
Q⁷ represents N or CH,
where at most five of the variables Q¹, Q², Q³, Q⁴, Q⁵, Q⁶ and Q⁷ simultaneously represent nitrogen and Q³ and Q⁵ do not simultaneously represent N and
R⁶ represents hydrogen, (C₁-C₄)-alkyl, (C₁-C₄)-haloalkyl, (C₁-C₄)-cyanoalkyl, (C₁-C₄)-alkoxy-(C₁-C₄)-alkyl, (C₁-C₄)-haloalkoxy-(C₁-C₄)-alkyl, (C₂-C₄)-alkenyl, (C₂-C₄)-alkenyloxy- (C₁-C₄)-alkyl, (C₂-C₄)-haloalkenyloxy-(C₁-C₄)-alkyl, (C₂-C₄)-haloalkenyl, (C₂-C₄)-cyanoalkenyl, (C₂-C₄)-alkynyl, (C₂-C₄)-alkynyloxy- (C₁-C₄)-alkyl, (C₂-C₄)-haloalkynyl, (C₃-C₆)-cycloalkyl, (C₃-C₆)-cycloalkyl-(C₃-C₆)-cycloalkyl, (C₁-C₄)-alkyl-(C₃-C₆)-cycloalkyl, halo- (C₃-C₆)-cycloalkyl, (C₁-C₄)-alkylthio-(C₁-C₄)-alkyl, (C₁-C₄)-alkylsulfinyl-(C₁-C₄)-alkyl, (C₁-C₄)-alkylsulfonyl-(C₁-C₄)-alkyl or (C₁-C₄)-alkylcarbonyl- (C₁-C₄)-alkyl and
R⁷ represents (C₁-C₄)-alkyl, (C₁-C₄)-haloalkyl, (C₁-C₄)-cyanoalkyl, (C₁-C₄)-alkoxy-(C₁-C₄)-alkyl, (C₁-C₄)-haloalkoxy-(C₁-C₄)-alkyl, (C₂-C₄)-alkenyl, (C₂-C₄)-alkenyloxy-(C₁-C₄)-alkyl, (C₂-C₄)-haloalkenyloxy-(C₁-C₄)-alkyl, (C₂-C₄)-haloalkenyl, (C₂-C₄)-cyanoalkenyl, (C₂-C₄)-alkynyl, (C₂-C₄)-alkynyloxy-(C₁-C₄)-alkyl, (C₂-C₄)-haloalkynyl, (C₃-C₆)-cycloalkyl, (C₃-C₆)-cycloalkyl- (C₃-C₆)-cycloalkyl, (C₁-C₄)-alkyl-(C₃-C₆)-cycloalkyl, halo-(C₃-C₆)-cycloalkyl, (C₁-C₄)-alkylthio-(C₁-C₄)-alkyl, (C₁-C₄)-alkylsulfinyl-(C₁-C₄)-alkyl, (C₁-C₄)-alkylsulfonyl-(C₁-C₄)-alkyl or (C₁-C₄)-alkylcarbonyl-(C₁-C₄)-alkyl,
A represents hydrogen, cyano, halogen, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₃-C₆)-cycloalkyl, (C₃-C₆)-cycloalkyl-(C₃-C₆)-cycloalkyl, (C₁-C₄)-alkyl-(C₃-C₆)-cycloalkyl, (C₁-C₄)-alkoxy, (C₁-C₄)-haloalkoxy, (C₁-C₄)-alkoxyimino, (C₁-C₄)-alkylthio, (C₁-C₄)-haloalkylthio, (C₁-C₄)-alkylsulfinyl, (C₁-C₄)-haloalkylsulfinyl, (C₁-C₄)-alkylsulfonyl, (C₁-C₄)-haloalkylsulfonyl, (C₁-C₄)-alkylsulfonyloxy, (C₁-C₄)-alkylcarbonyl, (C₁-C₄)-haloalkylcarbonyl, aminocarbonyl, (C₁-C₄)-alkylaminocarbonyl, di-(C₁-C₄)-alkylaminocarbonyl, (C₁-C₄)-alkylsulfonylamino, (C₁-C₄)-alkylamino, di-(C₁-C₄)-alkylamino, aminosulfonyl, (C₁-C₄)-alkylaminosulfonyl or di-(C₁-C₄)-alkylaminosulfonyl, or
A represents -O-CF₂-O- and, together with Q¹ and the carbon atom to which it is attached, forms a five-membered ring where Q¹ represents carbon,
W represents halogen or S(O)ₙR⁸, where
R⁸ represents (C₁-C₆)-alkyl, (C₁-C₆)-cyanoalkyl, (C₁-C₆)-alkoxy-(C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl or (C₃-C₈)-cycloalkyl and
n represents 0, 1 or 2,
V represents (C₁-C₆)-alkyl, (C₁-C₆)-haloalkyl, (C₁-C₆)-cyanoalkyl, (C₁-C₆)-alkoxy-(C₁-C₆)-alkyl, (C₁-C₆)-haloalkoxy-(C₁-C₆)-alkyl, (C₂-C₆)-alkenyl, (C₂-C₆)-alkenyloxy-(C₁-C₆)-alkyl, (C₂-C₆)-haloalkenyloxy-(C₁-C₆)-alkyl, (C₂-C₆)-haloalkenyl, (C₂-C₆)-cyanoalkenyl, (C₂-C₆)-alkynyl, (C₂-C₆)-alkynyloxy-(C₁-C₆)-alkyl, (C₂-C₆)-haloalkynyloxy-(C₁-C₆)-alkyl, (C₂-C₆)-haloalkynyl, (C₂-C₆)-cyanoalkynyl, (C₃-C₈)-cycloalkyl, (C₃-C₈)-cycloalkyl-(C₃-C₈)-cycloalkyl, (C₁-C₆)-alkyl-(C₃-C₈)-cycloalkyl, halo-(C₃-C₈)-cycloalkyl, cyano-(C₃-C₈)-cycloalkyl, (C₁-C₆)-alkylthio-(C₁-C₆)-alkyl, (C₁-C₆)-haloalkylthio-(C₁-C₆)-alkyl, (C₁-C₆)-alkylsulfinyl-(C₁-C₆)-alkyl, (C₁-C₆)-haloalkylsulfinyl-(C₁-C₆)-alkyl, (C₁-C₆)-alkylsulfonyl-(C₁-C₆)-alkyl, (C₁-C₆)-haloalkylsulfonyl-(C₁-C₆)-alkyl, (C₁-C₆)-alkylcarbonyl-(C₁-C₆)-alkyl, (C₁-C₆)-haloalkylcarbonyl-(C₁-C₆)-alkyl, (C₁-C₆)-alkoxycarbonyl-(C₁-C₆)-alkyl or (C₁-C₆)-haloalkoxycarbonyl-(C₁-C₆)-alkyl and
Y represents halogen, cyano, nitro, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-haloalkylthio, (C₁-C₄)-alkylsulfinyl, (C₁-C₄)-haloalkylsulfinyl, (C₁-C₄)-alkylsulfonyl, (C₁-C₄)-haloalkylsulfonyl, SCN, (C₁-C₄)-alkylcarbonyl, (C₁-C₄)-haloalkylcarbonyl, (C₁-C₄)-alkoxycarbonyl, (C₁-C₄)-haloalkoxycarbonyl, aminocarbonyl, (C₁-C₄)-alkylaminocarbonyl, di-(C₁-C₄)-alkylaminocarbonyl, (C₁-C₄)-haloalkylaminocarbonyl, (C₃-C₆)-cycloalkylaminocarbonyl, aminothiocarbonyl, (C₁-C₄)-alkylaminothiocarbonyl, di-(C₁-C₄)-alkylaminothiocarbonyl, (C₁-C₄)-haloalkylaminothiocarbonyl, (C₃-C₆)-cycloalkylaminothiocarbonyl, amino, (C₁-C₄)-alkylamino, (C₁-C₄)-haloalkylamino, di-(C₁-C₄)-alkylamino, (C₃-C₆)-cycloalkylamino, (C₁-C₄)-alkylsulfonylamino, (C₁-C₄)-alkylcarbonylamino, (C₁-C₄)-haloalkylcarbonylamino, (C₁-C₄)-alkylcarbonyl-(C₁-C₄)-alkylamino, (C₁-C₄)-haloalkylcarbonyl-(C₁-C₄)-alkylamino, (C₃-C₆)-cycloalkylcarbonylamino, (C₃-C₆)-cycloalkylcarbonyl-(C₁-C₄)-alkylamino, (C₁-C₄)-alkylthiocarbonylamino, (C₁-C₄)-haloalkylthiocarbonylamino, (C₁-C₄)-alkylthiocarbonyl-(C₁-C₄)-alkylamino, (C₁-C₄)-haloalkylthiocarbonyl-(C₁-C₄)-alkylamino, (C₃-C₆)-cycloalkylthiocarbonylamino, (C₃-C₆)-cycloalkylthiocarbonyl-(C₁-C₄)-alkylamino, (C₂-C₄)-alkenyl, (C₂-C₄)-haloalkenyl, (C₂-C₄)-cyanoalkenyl, (C₃-C₆)-cycloalkyl-(C₂)-alkenyl, (C₂-C₄)-alkynyl or (C₂-C₄)-haloalkynyl,
or represents (C₃-C₆)-cycloalkyl or (C₅-C₆)-cycloalkenyl, each of which is optionally mono- or polysubstituted by identical or different substituents, possible substituents being in each case: (C₃-C₆)-cycloalkyl, (C₁-C₄)-alkyl, (C₁-C₄)-haloalkyl, (C₁-C₄)-alkoxy, (C₁-C₄)-haloalkoxy, aminocarbonyl, aminothiocarbonyl, halogen or cyano,
or represents aryl or hetaryl, each of which is optionally mono- or polysubstituted by identical or different substituents, where (in the case of hetaryl) optionally at least one carbonyl group may be present and where possible substituents in each case are: cyano, carboxyl, halogen, nitro, acetyl, hydroxy, amino, SCN, SF₅, tri-(C₁-C₄)-alkylsilyl, (C₃-C₆)-cycloalkyl, (C₃-C₆)-cycloalkyl- (C₃-C₆)-cycloalkyl, (C₁-C₄)-alkyl- (C₃-C₆)-cycloalkyl, (C₁-C₄)-haloalkyl-(C₃-C₆)-cycloalkyl, halo-(C₃-C₆)-cycloalkyl, cyano-(C₃-C₆)-cycloalkyl, (C₁-C₄)-alkyl, (C₁-C₄)-haloalkyl, (C₁-C₄)-cyanoalkyl, (C₁-C₄)-hydroxyalkyl, hydroxycarbonyl-(C₁-C₄)-alkoxy, (C₁-C₄)-alkoxycarbonyl- (C₁-C₄)-alkyl, (C₁-C₄)-alkoxy- (C₁-C₄)-alkyl, (C₂-C₄)-alkenyl, (C₂-C₄)-haloalkenyl, (C₂-C₄)-cyanoalkenyl, (C₃-C₆)-cycloalkyl-(C₂)-alkenyl, (C₂-C₄)-alkynyl, (C₂-C₄)-haloalkynyl, (C₂-C₄)-cyanoalkynyl, (C₁-C₄)-alkoxy, (C₁-C₄)-haloalkoxy, (C₁-C₄)-cyanoalkoxy, (C₁-C₄)-alkoxycarbonyl-(C₁-C₄)-alkoxy, (C₁-C₄)-alkoxy-(C₁-C₄)-alkoxy, (C₁-C₄)-alkoxyimino, (C₁-C₄)-haloalkoxyimino, (C₁-C₄)-alkylthio, (C₁-C₄)-haloalkylthio, (C₁-C₄)-alkoxy- (C₁-C₄)-alkylthio, (C₁-C₄)-alkylthio-(C₁-C₄)-alkyl, (C₁-C₄)-alkylsulfinyl, (C₁-C₄)-haloalkylsulfinyl, (C₁-C₄)-alkoxy-(C₁-C₄)-alkylsulfinyl, (C₁-C₄)-alkylsulfinyl-(C₁-C₄)-alkyl, (C₁-C₄)-alkylsulfonyl, (C₁-C₄)-haloalkylsulfonyl, (C₁-C₄)-alkoxy-(C₁-C₄)-alkylsulfonyl, (C₁-C₄)-alkylsulfonyl-(C₁-C₄)-alkyl, (C₁-C₄)-alkylsulfonyloxy, (C₁-C₄)-haloalkylsulfonyloxy, (C₁-C₄)-alkylcarbonyl, (C₁-C₄)-haloalkylcarbonyl, (C₁-C₄)-alkylcarbonyloxy, (C₁-C₄)-alkoxycarbonyl, (C₁-C₄)-haloalkoxycarbonyl, aminocarbonyl, (C₁-C₄)-alkylaminocarbonyl, (C₁-C₄)-haloalkylaminocarbonyl, di-(C₁-C₄)-alkylaminocarbonyl, (C₂-C₄)-alkenylaminocarbonyl, di-(C₂-C₄)-alkenylaminocarbonyl, (C₃-C₆)-cycloalkylaminocarbonyl, (C₁-C₄)-alkylsulfonylamino, (C₁-C₄)-alkylamino, di-(C₁-C₄)-alkylamino, (C₁-C₄)-haloalkylamino, (C₃-C₆)-cycloalkylamino, aminosulfonyl, (C₁-C₄)-alkylaminosulfonyl, di-(C₁-C₄)-alkylaminosulfonyl, (C₁-C₄)-alkylsulfoximino, aminothiocarbonyl, (C₁-C₄)-alkylaminothiocarbonyl, di-(C₁-C₄)-alkylaminothiocarbonyl, (C₁-C₄)-haloalkylaminothiocarbonyl, (C₃-C₆)-cycloalkylaminothiocarbonyl, (C₁-C₄)-alkylcarbonylamino, (C₁-C₄)-haloalkylcarbonylamino, (C₁-C₄)-alkylcarbonyl-(C₁-C₄)-alkylamino, (C₁-C₄)-haloalkylcarbonyl-(C₁-C₄)-alkylamino, (C₃-C₆)-cycloalkylcarbonylamino, (C₃-C₆)-cycloalkylcarbonyl- (C₁-C₄)-alkylamino, (C₁-C₄)-alkylthiocarbonylamino, (C₁-C₄)-haloalkylthiocarbonylamino, (C₁-C₄)-alkylthiocarbonyl-(C₁-C₄)-alkylamino, (C₁-C₄)-haloalkylthiocarbonyl-(C₁-C₄)-alkylamino, (C₃-C₆)-cycloalkylthiocarbonylamino, (C₃-C₆)-cycloalkylthiocarbonyl-(C₁-C₄)-alkylamino, hetaryl, oxohetaryl, halohetaryl, halooxohetaryl, cyanohetaryl, cyanooxohetaryl, (C₁-C₄)-haloalkylhetaryl or (C₁-C₄)-haloalkyloxohetaryl,
**characterized in that**, in a first process step a), a compound of the formula (I) 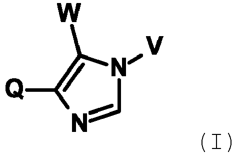
in which Q, V and W each have the meanings given above, is reacted with an organozinc base of the structure (NR^{a}R^{b})-Zn-R^{c} or (NR^{a}R^{b})₂-Zn, in which
R^{c} represents halogen or -O-pivaloyl and
R^{a} and R^{b} together form a -(CH₂)₄-, - (CH₂)₅- or - (CH₂)₂O(CH₂)₂- group, where each of these groups may optionally be substituted by 1, 2, 3 or 4 R^{d} radicals and R^{d} is selected from the group consisting of methyl, ethyl, n-propyl and isopropyl,
to give a compound of the formula (IVa) or the formula (IVb), 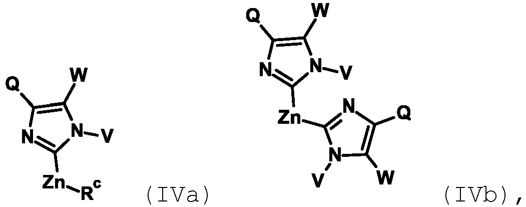
in which Q, V, W and R^{c} each have the meanings given above,
and this compound of the formula (IVa) or (IVb) is reacted in a second process step b) with a compound of the structure Y-X, in which X represents halogen and Y has the meaning given above, to give the compound of the formula (II).

2. Process according to Claim 1, **characterized in that** Q represents a structural element from the group of Q1 to Q14, 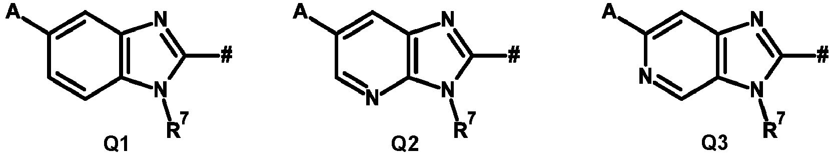 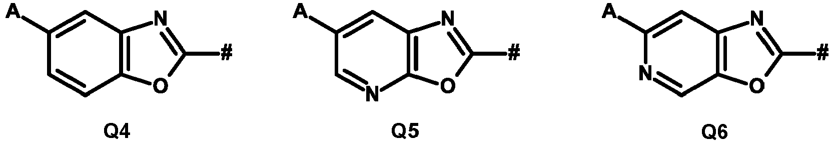 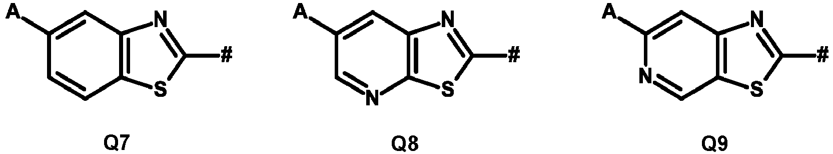 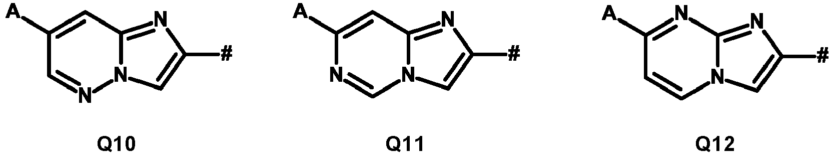 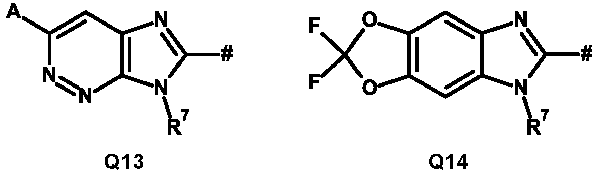 where
R⁷ represents (C₁-C₄)-alkyl, (C₁-C₄)-haloalkyl, (C₁-C₄)-alkoxy- (C₁-C₄)-alkyl, (C₁-C₄)-haloalkoxy-(C₁-C₄)-alkyl, (C₁-C₄)-alkylthio-(C₁-C₄)-alkyl, (C₁-C₄)-alkylsulfinyl-(C₁-C₄)-alkyl, (C₁-C₄)-alkylsulfonyl-(C₁-C₄)-alkyl or (C₁-C₄)-alkylcarbonyl-(C₁-C₄)-alkyl and
A represents fluorine, chlorine, bromine, fluoromethyl, difluoromethyl, trifluoromethyl, fluoroethyl (CH₂CFH₂, CHFCH₃), difluoroethyl (CF₂CH₃, CH₂CHF₂, CHFCFH₂), trifluoroethyl, (CH₂CF₃, CHFCHF₂, CF₂CFH₂), tetrafluoroethyl (CHFCF₃, CF₂CHF₂), pentafluoroethyl, trifluoromethoxy, difluorochloromethoxy, dichlorofluoromethoxy, trifluoromethylthio, trifluoromethylsulfinyl or trifluoromethylsulfonyl,
W represents halogen or S(O)ₙR⁸, where
R⁸ represents (C₁-C₆) -alkyl, (C₁-C₆) -cyanoalkyl, (C₁-C₆) -alkoxy- (C₁-C₆) -alkyl, (C₁-C₆) -haloalkyl, (C₂-C₆) - alkenyl, (C₂-C₆) -haloalkenyl, (C₂-C₆) -alkynyl, (C₂-C₆) - haloalkynyl or (C₃-C₈)-cycloalkyl and
n represents 0, 1 or 2,
R^{c} represents halogen, in particular chlorine, bromine or iodine,
V represents (C₁-C₆) -alkyl, (C₁-C₆) -cyanoalkyl, (C₁-C₆) -alkoxy- (C₁-C₆) -alkyl, (C₁-C₆) -haloalkyl, (C₂-C₆)-alkenyl, (C₂-C₆) -haloalkenyl, (C₂-C₆) -alkynyl, (C₂-C₆) - haloalkynyl or (C₃-C₈)-cycloalkyl and
Y represents halogen, cyano, (C₁-C₄) -alkyl, (C₁-C₄)-haloalkyl, (C₁-C₄) -alkoxy, (C₁-C₄) -haloalkoxy, aminocarbonyl, (C₁-C₄) -alkylaminocarbonyl, di-(C₁-C₄)-alkylaminocarbonyl, (C₁-C₄)-haloalkylaminocarbonyl, (C₃-C₆)-cycloalkylaminocarbonyl, amino, (C₁-C₄)-alkylamino, (C₁-C₄) -haloalkylamino, di-(C₁-C₄) -alkylamino, (C₃-C₆)-cycloalkylamino, (C₁-C₄) -alkylsulfonylamino, (C₁-C₄) - alkylcarbonylamino, (C₁-C₄)-haloalkylcarbonylamino, (C₁-C₄) -alkylcarbonyl- (C₁-C₂) -alkylamino, (C₁-C₄) - haloalkylcarbonyl- (C₁-C₂) -alkylamino, (C₃-C₆)-cycloalkylcarbonylamino, (C₃-C₆)-cycloalkylcarbonyl-(C₁-C₂) -alkylamino, (C₂-C₄) -alkenyl, (C₂-C₄) -haloalkenyl, (C₂-C₄)-cyanoalkenyl or (C₃-C₆)-cycloalkyl- (C₂) -alkenyl,
or represents (C₃-C₆) -cycloalkyl or (C₅-C₆) -cycloalkenyl, each of which is optionally mono- or polysubstituted by identical or different substituents, possible substituents being in each case: (C₃-C₆)-cycloalkyl, (C₁-C₄) -alkyl, (C₁-C₄) -haloalkyl, (C₁-C₄) -alkoxy, (C₁-C₄)-haloalkoxy, halogen or cyano,
or represents phenyl, pyridyl, pyrimidyl, pyridazinyl, thiophenyl, furanyl, pyrazolyl, pyrrolyl, thiazolyl, oxazolyl or imidazolyl, each of which is optionally mono-or polysubstituted by identical or different substituents, possible substituents being in each case: cyano, halogen, nitro, acetyl, hydroxy, amino, SF₅-, (C₃-C₆) -cycloalkyl, (C₁-C₄) -alkyl- (C₃-C₆)-cycloalkyl, halo-(C₃-C₆)-cycloalkyl, (C₁-C₄) -alkyl, (C₁-C₄) -haloalkyl, (C₁-C₄) -cyanoalkyl, (C₁-C₄) -hydroxyalkyl, (C₁-C₄) -alkoxy- (C₁-C₂) -alkyl, (C₂-C₄) -alkenyl, (C₂-C₄) -haloalkenyl, (C₂-C₄)-cyanoalkenyl, (C₃-C₆)-cycloalkyl- (C₂) -alkenyl, (C₂-C₄)-alkynyl, (C₂-C₄) -haloalkynyl, (C₂-C₄) -cyanoalkynyl, (C₁-C₄) -alkoxy, (C₁-C₄) -haloalkoxy, (C₁-C₄) -cyanoalkoxy, (C₁-C₄) -alkoxy- (C₁-C₂) -alkoxy, (C₁-C₄) -alkoxyimino, (C₁-C₄)-haloalkoxyimino, (C₁-C₄) -alkylthio, (C₁-C₄)-haloalkylthio, (C₁-C₄) -alkylthio- (C₁-C₂) -alkyl, (C₁-C₄)-alkylsulfinyl, (C₁-C₄) -haloalkylsulfinyl, (C₁-C₄)-alkylsulfonyl, (C₁-C₄) -haloalkylsulfonyl, (C₁-C₄)-alkylsulfonyloxy, (C₁-C₄) -haloalkylsulfonyloxy, (C₁-C₄)-alkylcarbonyl, (C₁-C₄)-haloalkylcarbonyl, aminocarbonyl, (C₁-C₄) -alkylaminocarbonyl, (C₁-C₄)-haloalkylaminocarbonyl, di-(C₁-C₄)-alkylaminocarbonyl, (C₃-C₆)-cycloalkylaminocarbonyl, aminothiocarbonyl, (C₁-C₄)-alkylaminothiocarbonyl, di-(C₁-C₄)-alkylaminothiocarbonyl, (C₁-C₄)-haloalkylaminothiocarbonyl, (C₃-C₆)-cycloalkylaminothiocarbonyl, (C₁-C₄)-alkylsulfonylamino, (C₁-C₄) -alkylamino, di-(C₁-C₄) -alkylamino, (C₁-C₄)-haloalkylamino, (C₃-C₆)-cycloalkylamino, aminosulfonyl, (C₁-C₄) -alkylaminosulfonyl, di-(C₁-C₄)-alkylaminosulfonyl, (C₁-C₄)-alkylcarbonylamino, (C₁-C₄)-haloalkylcarbonylamino, (C₁-C₄) -alkylcarbonyl- (C₁-C₂)-alkylamino, (C₁-C₂) -haloalkylcarbonyl- (C₁-C₂) -alkylamino, (C₃-C₆)-cycloalkylcarbonylamino, (C₃-C₆)-cycloalkylcarbonyl- (C₁-C₂) -alkylamino, (C₁-C₄)-alkylthiocarbonylamino, (C₁-C₄)-haloalkylthiocarbonylamino, (C₁-C₄)-alkylthiocarbonyl-(C₁-C₂) -alkylamino, (C₁-C₄) -haloalkylthiocarbonyl- (C₁-C₂)-alkylamino, (C₃-C₆)-cycloalkylthiocarbonylamino or (C₃-C₆)-cycloalkylthiocarbonyl- (C₁-C₂) -alkylamino.

3. Process according to Claim 1, **characterized in that** Q represents a structural element from the group of Q2, Q3, Q4, Q10, Q11, Q13 or Q14, where
R⁷ represents (C₁-C₄) -alkyl or (C₁-C₄) -alkoxy- (C₁-C₄)-alkyl and
A represents trifluoromethyl, fluoroethyl (CH₂CFH₂, CHFCH₃), difluoroethyl (CF₂CH₃, CH₂CHF₂, CHFCFH₂), trifluoroethyl, (CH₂CF₃, CHFCHF₂, CF₂CFH₂), tetrafluoroethyl (CHFCF₃, CF₂CHF₂), pentafluoroethyl, trifluoromethylthio, trifluoromethylsulfinyl or trifluoromethylsulfonyl,
W represents halogen or S(O)R^{B}, where
R⁸ represents methyl, ethyl, n-propyl or isopropyl and
n represents 0, 1 or 2,
R^{c} represents chlorine,
V represents methyl, ethyl, n-propyl or isopropyl and Y represents bromine, iodine, cyano, ethenyl, cyclopropylethenyl, isopropenyl, cyclopropylethynyl, methyl, ethyl, isopropyl, cyclopropylethyl, methoxycarbonyl, trifluoroethylaminocarbonyl, aminocarbonyl, methylaminocarbonyl, dimethylaminocarbonyl, ethylaminocarbonyl, aminothiocarbonyl, methylaminothiocarbonyl, dimethylaminothiocarbonyl,
or represents cyclopropyl, cyclobutyl, cyclopentyl, cyclohexyl, cyclopentenyl or cyclohexenyl, each of which is optionally mono- or disubstituted by identical or different substituents, possible substituents being in each case: methyl, ethyl, n-propyl, isopropyl, cyclopropyl, difluoromethyl, trifluoromethyl, cyano, fluorine or chlorine,
or represents phenyl, pyridin-2-yl, pyridin-3-yl, pyridin-4-yl, pyrimidin-5-yl, pyridazin-3-yl, pyridazin-4-yl, thien-2-yl, thien-3-yl, 1,3-thiazol-5-yl, 1H-imidazol-1-yl, 1H-imidazol-2-yl, 1H-imidazol-5-yl, 1H-pyrazol-1-yl, 1H-pyrazol-3-yl, 1H-pyrazol-4-yl, 1H-pyrazol-5-yl, 1H-pyrrol-1-yl, 1H-pyrrol-2-yl, 1H-pyrrol-3-yl or 1-cyclohexenyl, each of which is optionally mono-, di- or trisubstituted by identical or different substituents, possible substituents being in each case: cyano, fluorine, chlorine, methyl, cyclopropyl, cyanomethyl, cyanoisopropyl, cyanocyclopropyl, trifluoromethyl, trifluoroethyl or aminocarbonyl.

4. Process according to Claim 1, **characterized in that**
Q represents the structural element Q3 or Q13, where
R⁷ represents methyl or ethyl, in particular methyl, and
A represents trifluoromethyl or pentafluoroethyl,
W represents S(O)ₙR⁸, where
R⁸ represents ethyl and
n represents 0 or 2,
R^{c} represents chlorine,
V represents methyl or ethyl, in particular methyl, and
Y represents bromine, 5-cyanopyridin-2-yl or 6-chloropyridazin-3-yl.

5. Process according to any of Claims 1 to 4, **characterized in that** the organozinc base is a compound of the formula (VI)
(VI) (TMP)ₓ ZnCl₂₋ₓ,
in which x represents the number 1 or 2.

6. Process according to any of Claims 1 to 5, **characterized in that** the organozinc base is present in conjunction with an alkali metal halide or alkaline earth metal halide, preferably lithium chloride and/or magnesium chloride.

7. Process according to any of Claims 1 to 6, **characterized in that** process step a) is carried out at a temperature between 0°C and 110°C.

8. Process according to any of Claims 1 to 7, **characterized in that** X represents bromine or iodine.

9. Process according to any of Claims 1 to 8, **characterized in that** Y represents halogen.

10. Process according to Claim 9, **characterized in that** the compound X-Y is an elemental halogen, especially F₂, Cl₂, Br₂ or I₂.

11. Process according to any of Claims 1 to 8, **characterized in that** Y is as defined according to any of Claims 1 to 4, but does not represent halogen.

12. Process according to either of Claims 9 and 10, **characterized in that** process step b) is carried out at a temperature between 0°C and 80°C.

13. Process according to Claim 11, **characterized in that** process step b) is carried out at a temperature between 0°C and 120°C.

14. Process according to any of Claims 1 to 13, **characterized in that** process step a) and process step b) are carried out in the presence of an organic solvent, where the solvent is selected from THF and N,N-dimethylformamide (DMF).

15. Compounds of the formula (IVa) 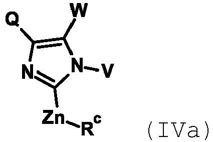 in which Q, V, R^{c} and W have the meanings according to any of Claims 1 to 4.

16. Compounds of the formula (IVb) 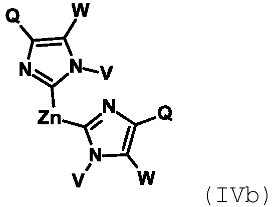 in which Q, V and W have the meanings according to any of Claims 1 to 4.

17. Compounds of the formula (IVa) according to Claim 15 or (IVb) according to Claim 16, **characterized in that** said compounds are present complexed with salts, where the salts are alkali metal halides or alkaline earth metal halides, preferably lithium chloride and/or magnesium chloride.

## Revendications

1. Procédé de préparation de composés de formule (II) 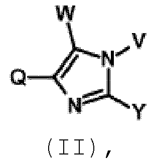 dans laquelle
Q représente un élément structural 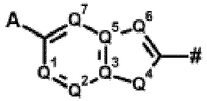
dans laquelle le signe # indique la liaison au reste de la molécule et
Q¹ représente N ou CR⁶,
Q² représente N ou CR⁶,
Q3 représente N ou C,
Q⁴ représente 0, S, N, CR⁶ ou NR⁷,
Q⁵ représente N ou C,
Q⁶ représente N ou CH et
Q⁷ représente N ou CH,
dans laquelle au maximum cinq des variables Q¹, Q², Q³, Q⁴, Q⁵ᵣ Q⁶ et Q⁷ représentent simultanément azote et Q³ et Q⁵ ne représentent pas simultanément N et
R⁶ représente hydrogène, (C₁-C₄) alkyle, (C₁-C₄) halogénoalkyle, (C₁-C₄) cyanoalkyle, (C₁-C₄)alcoxy-(C₁-C₄) alkyle, (C₁-C₄) halogénoalcoxy- (C₁-C₄) alkyle, (C₂-C₄)alcényle, (C₂-C₄) alcényloxy-(C₁-C₄) alkyle, (C₂-C₄)halogénoalcényloxy-(C₁-C₄) alkyle, (C₂-C₄) halogénoalcényle, (C₂-C₄) cyanoalcényle, (C₂-C₄) alcynyle, (C₂-C₄) alcynyloxy- (C₁-C₄) alkyle, (C₂-C₄) halogénoalcynyle, (C₃-C₆)cycloalkyle, (C₃-C₆) cycloalkyl- (C₃-C₆)cycloalkyle, (C₁-C₄) alkyl- (C₃-C₆) cycloalkyle, halogéno (C₃-C₆)cycloalkyle, (C₁-C₄) alkylthio-(C₁-C₄) alkyle, (C₁-C₄) alkylsulfinyl-(C₁-C₄) alkyle, (C₁-C₄) alkylsulfonyl- (C₁-C₄) alkyle ou (C₁-C₄) alkylcarbonyl-(C₁-C₄) alkyle et
R⁷ représente (C₁-C₄) alkyle, (C₁-C₄) halogénoalkyle, (C₁-C₄) cyanoalkyle, (C₁-C₄) alcoxy- (C₁-C₄) alkyle, (C₁-C₄) halogénoalcoxy- (C₁-C₄) alkyle, (C₂-C₄) alcényle, (C₂-C₄) alcényloxy- (C₁-C₄) alkyle, (C₂-C₄) halogénoalcényloxy-(C₁-C₄) alkyle, (C₂-C₄) halogénoalcényle, (C₂-C₄) cyanoalcényle, (C₂-C₄) alcynyle, (C₂-C₄) alcynyloxy- (C₁-C₄) alkyle, (C₂-C₄) halogénoalcynyle, (C₃-C₆)cycloalkyle, (C₃-C₆)cycloalkyl- (C₃-C₆)cycloalkyle, (C₁-C₄) alkyl- (C₃-C₆) cycloalkyle, halogéno (C₃-C₆)cycloalkyle, (C₁-C₄) alkylthio-(C₁-C₄) alkyle, (C₁-C₄) alkylsulfinyl-(C₁-C₄) alkyle, (C₁-C₄) alkylsulfonyl- (C₁-C₄) alkyle ou (C₁-C₄) alkylcarbonyl-(C₁-C₄) alkyle,
A représente hydrogène, cyano, halogène, (C₁-C₄) alkyle, (C₁-C₄) halogénoalkyle, (C₂-C₄) alcényle, (C₂-C₄) halogénoalcényle, (C₂-C₄) alcynyle, (C₂-C₄) halogénoalcynyle, (C₃-C₆)cycloalkyle, (C₃-C₆) cycloalkyl- (C₃-C₆)cycloalkyle, (C₁-C₄) alkyl- (C₃-C₆) cycloalkyle, (C₁-C₄) alcoxy, (C₁-C₄) halogénoalcoxy, (C₁-C₄) alcoxyimino, (C₁-C₄) alkylthio, (C₁-C₄) halogénoalkylthio, (C₁-C₄) alkylsulfinyle, (C₁-C₄) halogénoalkylsulfinyle, (C₁-C₄) alkylsulfonyle, (C₁-C₄)halogénoalkylsulfonyle, (C₁-C₄) alkylsulfonyloxy, (C₁-C₄)alkylcarbonyle, (C₁-C₄) halogénoalkylcarbonyle, aminocarbonyle, (C₁-C₄) alkylaminocarbonyle, di-(C₁-C₄) alkyl-aminocarbonyle, (C₁-C₄) alkylsulfonylamino, (C₁-C₄) alkylamino, di-(C₁-C₄) alkylamino, aminosulfonyle, (C₁-C₄) alkylaminosulfonyle ou di-(C₁-C₄)alkylaminosulfonyle, ou
A représente -O-CF₂-O- et forme, conjointement avec Q¹ et l'atome de carbone auquel il est lié, un cycle à cinq chaînons, dans lequel Q¹ représente carbone,
W représente halogène ou S(O)R⁸, dans lequel R⁸ représente (C₁-C₆) alkyle, (C₁-C₆) cyanoalkyle, (C₁-C₆) alcoxy- (C₁-Ce) alkyle, (C₁-C₆) halogénoalkyle, (C₂-C₆) alcényle, (C₂-C₆) halogénoalcényle, (C₂-C₆) alcynyle, (C₂-C₆)halogénoalcynyle ou (C₃-C₈)cycloalkyle et
n représente 0, 1 ou 2,
V représente (C₁-C₆)-alkyle, (C₁-C₆) halogénoalkyle, (C₁-C₆) cyanoalkyle, (C₁-C₆) alcoxy-(C₁-C₆) alkyle, (C₁-C₆) halogénoalcoxy- (C₁-C₆) alkyle, (C₂-C₆) alcényle, (C₂-C₆) alcényloxy- (C₁-C₆) alkyle, (C₂-C₆) halogénoalcényloxy-(C₁-C₆) alkyle, (C₂-C₆) halogénoalcényle, (C₂-C₆) cyanoalcényle, (C₂-C₆) alcynyle, (C₂-C₆) alcynyloxy- (C₁-C₆) alkyle, (C₂-C₆) halogénoalcynyloxy- (C₁-C₆) alkyle, (C₂-C₆) halogénoalcynyle, (C₂-C₆) cyanoalcynyle, (C₃-C₈) cycloalkyle, (C₃-C₈) cycloalkyl- (C₃-C₈) cycloalkyle, (C₁-C₆) alkyl- (C₃-C₈) cycloalkyle, halogéno (C₃-C₈) cycloalkyle, cyano (C₃-C₈) cycloalkyle, (C₁-C₆) alkylthio- (C₁-C₆) alkyle, (C₁-C₆) halogénoalkylthio- (C₁-C₆) alkyle, (C₁-C₆) alkylsulfinyl- (C₁-C₆) alkyle, (C₁-C₆) halogénoalkylsulfinyl- (C₁-C₆) alkyle, (C₁-C₆) alkylsulfonyl- (C₁-C₆) alkyle, (C₁-C₆) halogénoalkylsulfonyl- (C₁-C₆) alkyle, (C₁-C₆) alkylcarbonyl-(C₁-C₆) alkyle, (C₁-C₆) halogénoalkylcarbonyl- (C₁-C₆) alkyle, (C₁-C₆) alcoxycarbonyl-(C₁-C₆) alkyle ou (C₁-C₆)halogénoalcoxycarbonyl-(C₁-C₆)alkyle et
Y représente halogène, cyano, nitro, (C₁-C₄) alkyle, (C₁-C₄) halogénoalkyle, (C₁-C₄) alcoxy, (C₁-C₄) halogénoalcoxy, (C₁-C₄) alkylthio, (C₁-C₄) halogénoalkylthio, (C₁-C₄)alkylsulfinyle, (C₁-C₄) halogénoalkylsulfinyle, (C₁-C₄) alkylsulfonyle, (C₁-C₄) halogénoalkylsulfonyle, SCN, (C₁-C₄) alkylcarbonyle, (C₁-C₄)halogénoalkylcarbonyle, (C₁-C₄) alcoxycarbonyle, (C₁-C₄) halogénoalcoxycarbonyle, aminocarbonyle, (C₁-C₄) alkylaminocarbonyle, di-(C₁-C₄) alkyl-aminocarbonyle, (C₁-C₄)halogénoalkylaminocarbonyle, (C₃-C₆)cycloalkylaminocarbonyle, aminothiocarbonyle, (C₁-C₄)alkylaminothiocarbonyle, di-(C₁-C₄)alkylaminothiocarbonyle, (C₁-C₄)halogénoalkylaminothiocarbonyle, (C₃-C₆)cycloalkylaminothiocarbonyle, amino, (C₁-C₄) alkylamino, (C₁-C₄) halogénoalkylamino, di-(C₁-C₄)alkylamino, (C₃-C₆)cycloalkylamino, (C₁-C₄)alkylsulfonylamino, (C₁-C₄)alkylcarbonylamino, (C₁-C₄)halogénoalkylcarbonylamino, (C₁-C₄)alkylcarbonyl- (C₁-C₄)alkyl-amino, (C₁-C₄) halogénoalkylcarbonyl-(C₁-C₄)alkyl-amino, (C₃-C₆)cycloalkylcarbonylamino, (C₃-C₆)cycloalkylcarbonyl-(C₁-C₄)alkyl-amino, (C₁-C₄)alkylthiocarbonylamino, (C₁-C₄)halogénoalkylthiocarbonylamino, (C₁-C₄)alkylthiocarbonyl-(C₁-C₄)alkyl-amino, (C₁-C₄)halogénoalkylthiocarbonyl-(C₁-C₄)alkyl-amino, (C₃-C₆)cycloalkylthiocarbonylamino, (C₃-C₆)cycloalkylthiocarbonyl-(C₁-C₄)alkyl-amino, (C₂-C₄)alcényle, (C₂-C₄) halogénoalcényle, (C₂-C₄)cyanoalcényle, (C₃-C₆)cycloalkyl- (C₂)alcényle, (C₂-C₄)alcynyle ou (C₂-C₄) halogénoalcynyle,
ou représente (C₃-C₆)cycloalkyle ou (C₅-C₆)cycloalcényle à chaque fois éventuellement monosubstitué ou polysubstitué, de manière identique ou différente, où les substituants peuvent à chaque fois être : (C₃-C₆) cycloalkyle, (C₁-C₄) alkyle, (C₁-C₄) halogénoalkyle, (C₁-C₄) alcoxy, (C₁-C₄) halogénoalcoxy, aminocarbonyle, aminothiocarbonyle, halogène ou cyano,
ou représente aryle ou hétaryle à chaque fois éventuellement monosubstitué ou polysubstitué, de manière identique ou différente, où (dans le cas d'hétaryle) éventuellement au moins un groupe carbonyle peut être contenu et où les substituants peuvent à chaque fois être : cyano, carboxyle, halogène, nitro, acétyle, hydroxy, amino, SCN, SF₅, tri-(C₁-C₄)alkylsilyle, (C₃-C₆)cycloalkyle, (C₃-C₆)cycloalkyl- (C₃-C₆)cycloalkyle, (C₁-C₄) alkyl- (C₃-C₆)cycloalkyle, (C₁-C₄) halogénoalkyl-(C₃-C₆) cycloalkyle, halogène-(C₃-C₆)cycloalkyle, cyano-(C₃-C₆) cycloalkyle, (C₁-C₄) alkyle, (C₁-C₄) halogénoalkyle, (C₁-C₄) cyanoalkyle, (C₁-C₄) hydroxyalkyle, Hydroxycarbonyl-(C₁-C₄) -alcoxy, (C₁-C₄) alcoxycarbonyl-(C₁-C₄) alkyle, (C₁-C₄) alcoxy- (C₁-C₄) alkyle, (C₂-C₄) alcényle, (C₂-C₄) halogénoalcényle, (C₂-C₄) cyanoalcényle, (C₃-C₆) cycloalkyl-(C₂) alcényle, (C₂-C₄) alcynyle, (C₂-C₄) halogénoalcynyle, (C₂-C₄) cyanoalcynyle, (C₁-C₄) alcoxy, (C₁-C₄) halogénoalcoxy, (C₁-C₄) cyanoalcoxy, (C₁-C₄) alcoxycarbonyl- (C₁-C₄) alcoxy, (C₁-C₄) alcoxy-(C₁-C₄) alcoxy, (C₁-C₄) alcoxyimino, (C₁-C₄)halogénoalcoxyimino, (C₁-C₄) alkylthio, (C₁-C₄) halogénoalkylthio, (C₁-C₄) alcoxy- (C₁-C₄) alkylthio, (C₁-C₄) alkylthio- (C₁-C₄) alkyle, (C₁-C₄) alkylsulfinyle, (C₁-C₄) halogénoalkylsulfinyle, (C₁-C₄) alcoxy- (C₁-C₄)alkylsulfinyle, (C₁-C₄) alkylsulfinyl-(C₁-C₄) alkyle, (C₁-C₄) alkylsulfonyle, (C₁-C₄) halogénoalkylsulfonyle, (C₁-C₄) alcoxy- (C₁-C₄) alkylsulfonyle, (C₁-C₄) alkylsulfonyl- (C₁-C₄) alkyle, (C₁-C₄) alkylsulfonyloxy, (C₁-C₄) halogénoalkylsulfonyloxy, (C₁-C₄) alkylcarbonyle, (C₁-C₄) halogénoalkylcarbonyle, (C₁-C₄) alkylcarbonyloxy, (C₁-C₄) alcoxycarbonyle, (C₁-C₄) halogénoalcoxycarbonyle, aminocarbonyle, (C₁-C₄) alkylaminocarbonyle, (C₁-C₄)halogénoalkylaminocarbonyle, di-(C₁-C₄)alkyl-aminocarbonyle, (C₂-C₄) alcénylaminocarbonyle, di-(C₂-C₄) - alcénylaminocarbonyle, (C₃-C₆)cycloalkylaminocarbonyle, (C₁-C₄) alkylsulfonylamino, (C₁-C₄) alkylamino, di-(C₁-C₄)alkylamino, (C₁-C₄)halogénoalkylamino, (C₃-C₆)cycloalkylamino, aminosulfonyle, (C₁-C₄)alkylaminosulfonyle, di-(C₁-C₄)alkylaminosulfonyle, (C₁-C₄) alkylsulfoximino, aminothiocarbonyle, (C₁-C₄)alkylaminothiocarbonyle, di-(C₁-C₄)alkylaminothiocarbonyle, (C₁-C₄)halogénoalkylaminothiocarbonyle, (C₃-C₆)cycloalkylaminothiocarbonyle, (C₁-C₄) alkylcarbonylamino, (C₁-C₄)halogénoalkylcarbonylamino, (C₁-C₄) alkylcarbonyl-(C₁-C₄)alkyl-amino, (C₁-C₄) halogénoalkylcarbonyl-(C₁-C₄)alkyl-amino, (C₃-C₆)cycloalkylcarbonylamino, (C₃-C₆)cycloalkylcarbonyl-(C₁-C₄)alkyl-amino, (C₁-C₄)alkylthiocarbonylamino, (C₁-C₄)halogénoalkylthiocarbonylamino, (C₁-C₄)alkylthiocarbonyl-(C₁-C₄) alkyl-amino, (C₁-C₄)halogénoalkylthiocarbonyl-(C₁-C₄)alkyl-amino, (C₃-C₆)cycloalkylthiocarbonylamino, (C₃-C₆)cycloalkylthiocarbonyl-(C₁-C₄)alkyl-amino, hétaryle, oxo-hétaryle, halogène-hétaryle, halogène-oxo-hétaryle, cyano-hétaryle, cyano-oxo-hétaryle, (C₁-C₄)halogénoalkyl-hétaryle ou (C₁-C₄) halogénoalkyl-oxo-hétaryle,
**caractérisé en ce que** dans une première étape de procédé a) un composé de formule (I) 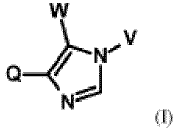
dans laquelle Q, V et W possèdent à chaque fois les significations mentionnées ci-dessus,
est transformé avec une base organométallique au zinc de structure (NR^{a}R^{b}) -Zn-R^{c} ou (NR^{a}R^{b}) ₂-Zn, dans lesquelles R^{c} représente halogène ou -O-pivaloyle et
R^{a} et R^{b} forment ensemble un groupe -(CH₂)₄-, -(CH₂)₅- ou - (CH₂)₂O(CH₂)₂, chacun de ces groupes pouvant être substitué par 1, 2, 3 ou 4 radicaux R^{d} et R^{d} étant choisi dans le groupe constitué par méthyle, éthyle, n-propyle et i-propyle,
pour donner un composé de formule (IVa) ou de formule (IVb), 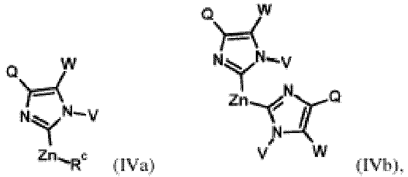
dans lesquelles Q, V, W et R^{c} possèdent à chaque fois les significations mentionnées ci-dessus,
et ce composé de formule (IVa) ou (IVb) est transformé dans une deuxième étape de procédé b) avec un composé de structure Y-X, dans laquelle X représente halogène et Y possède la signification mentionnée ci-dessus, pour donner un composé de formule (II).

2. procédé selon la revendication 1, **caractérisé en ce que**
Q représente un élément structural de la série Q¹ à Q14, 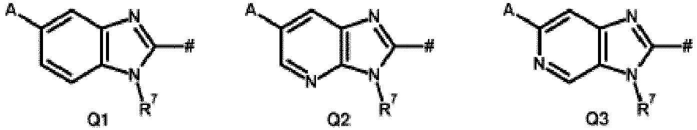 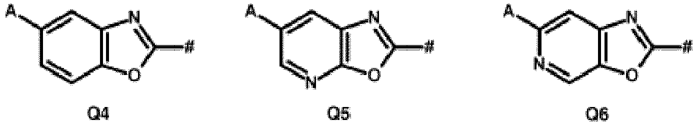 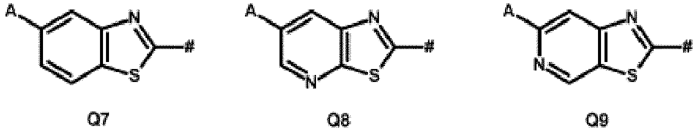 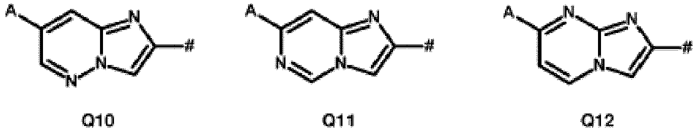 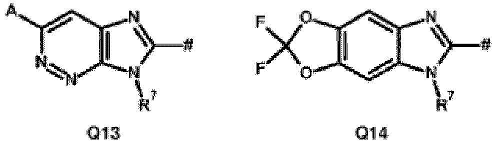 dans lesquels
R⁷ représente (C₁-C₄) alkyle, (C₁-C₄) halogénoalkyle, (C₁-C₄) alcoxy- (C₁-C₄) alkyle, (C₁-C₄) halogénoalcoxy- (C₁-C₄) alkyle, (C₁-C₄) alkylthio- (C₁-C₄) alkyle, (C₁-C₄)alkylsulfinyl-(C₁-C₄) alkyle, (C₁-C₄) alkylsulfonyl- (C₁-C₄) alkyle ou (C₁-C₄) alkylcarbonyl- (C₁-C₄) alkyle et
A représente fluor, chlore, brome, fluorométhyle, difluorométhyle, trifluorométhyle, fluoroéthyle (CH₂CFH₂, CHFCH₃), difluoroéthyle (CF₂CH₃, CH₂CHF₂, CHFCFH₂), trifluoroéthyle, (CH₂CF₃, CHFCHF₂, CF₂CFH₂), tétrafluoroéthyle (CHFCF₃, CF₂CHF₂), pentafluoroéthyle, trifluorométhoxy, difluorchlorméthoxy, dichlorfluorométhoxy, trifluorométhylthio, trifluorométhylsulfinyle ou trifluorométhylsulfonyle, W représente halogène ou S(O)ₙR⁸, dans lequel
R⁸ représente (C₁-Ce) alkyle, (C₁-Ce) cyanoalkyle, (C₁-C₆) alcoxy- (C₁-Ce) alkyle, (C₁-C₆) halogénoalkyle, (C₂-C₆) alcényle, (C₂-C₆) halogénoalcényle, (C₂-C₆) alcynyle, (C₂-C₆)halogénoalcynyle ou (C₃-C₈)cycloalkyle et
n représente 0, 1 ou 2,
R^{c} représente halogène, en particulier chlore, brome ou iode,
V représente (C₁-C₆) -alkyle, (C₁-C₆) cyanoalkyle, (C₁-C₆) alcoxy- (C₁-Ce) alkyle, (C₁-C₆) halogénoalkyle, (C₂-C₆) alcényle, (C₂-C₆) halogénoalcényle, (C₂-C₆) alcynyle, (C₂-C₆)halogénoalcynyle ou (C₃-C₈)cycloalkyle et
Y représente halogène, cyano, (C₁-C₄) alkyle, (C₁-C₄) halogénoalkyle, (C₁-C₄) alcoxy, (C₁-C₄) halogénoalcoxy, aminocarbonyle, (C₁-C₄) alkylaminocarbonyle, di-(C₁-C₄) alkyl-aminocarbonyle, (C₁-C₄)halogénoalkylaminocarbonyle, (C₃-C₆)cycloalkylaminocarbonyle, amino, (C₁-C₄) alkylamino, (C₁-C₄) halogénoalkylamino, di-(C₁-C₄) alkylamino, (C₃-C₆) cycloalkylamino, (C₁-C₄) alkylsulfonylamino, (C₁-C₄) alkylcarbonylamino, (C₁-C₄)halogénoalkylcarbonylamino, (C₁-C₄) alkylcarbonyl-(C₁-C₂)alkyl-amino, (C₁-C₄) halogénoalkylcarbonyl-(C₁-C₂)alkyl-amino, (C₃-C₆)cycloalkylcarbonylamino, (C₃-C₆)cycloalkylcarbonyl-(C₁-C₂) alkyl-amino, (C₂-C₄)alcényle, (C₂-C₄) halogénoalcényle, (C₂-C₄)cyanoalcényle ou (C₃-C₆)cycloalkyl-(C₂) alcényle,
ou représente (C₃-C₆)cycloalkyle ou (C₅-C₆)cycloalcényle à chaque fois éventuellement monosubstitué ou polysubstitué, de manière identique ou différente, où les substituants peuvent à chaque fois être : (C₃-C₆) cycloalkyle, (C₁-C₄) alkyle, (C₁-C₄) halogénoalkyle, (C₁-C₄)alcoxy, (C₁-C₄)halogénoalcoxy, halogène ou cyano,
ou représente phényle, pyridinyle, pyrimidinyle, pyridazinyle, thiophényle, furannyle, pyrazolyle, pyrrolyle, thiazolyle, oxazolyle ou imidazolyle, à chaque fois éventuellement monosubstitué ou polysubstitué, de manière identique ou différente, où les substituants peuvent à chaque fois être : cyano, halogène, nitro, acétyle, hydroxy, amino, SF₅-, (C₃-C₆)cycloalkyle, (C₁-C₄) alkyl- (C₃-C₆)cycloalkyle, halogéno (C₃-C₆)cycloalkyle, (C₁-C₄) alkyle, (C₁-C₄) halogénoalkyle, (C₁-C₄) cyanoalkyle, (C₁-C₄) hydroxyalkyle, (C₁-C₄) alcoxy- (C₁-C₂) alkyle, (C₂-C₄) alcényle, (C₂-C₄) halogénoalcényle, (C₂-C₄) cyanoalcényle, (C₃-C₆)cycloalkyl-(C₂) alcényle, (C₂-C₄) alcynyle, (C₂-C₄) halogénoalcynyle, (C₂-C₄) cyanoalcynyle, (C₁-C₄) alcoxy, (C₁-C₄) halogénoalcoxy, (C₁-C₄) cyanoalcoxy, (C₁-C₄) alcoxy- (C₁-C₂) alcoxy, (C₁-C₄) alcoxyimino, (C₁-C₄) halogénoalcoxyimino, (C₁-C₄) alkylthio, (C₁-C₄) halogénoalkylthio, (C₁-C₄) alkylthio-(C₁-C₂) alkyle, (C₁-C₄) alkylsulfinyle, (C₁-C₄)halogénoalkylsulfinyle, (C₁-C₄) alkylsulfonyle, (C₁-C₄)halogénoalkylsulfonyle, (C₁-C₄) alkylsulfonyloxy, (C₁-C₄)halogénoalkylsulfonyloxy, (C₁-C₄) alkylcarbonyle, (C₁-C₄)halogénoalkylcarbonyle, aminocarbonyle, (C₁-C₄) alkylaminocarbonyle, (C₁-C₄)halogénoalkylaminocarbonyle, di-(C₁-C₄) alkyl-aminocarbonyle, (C₃-C₆)cycloalkylaminocarbonyle, aminothiocarbonyle, (C₁-C₄)alkylaminothiocarbonyle, di-(C₁-C₄) alkylaminothiocarbonyle, (C₁-C₄)halogénoalkylaminothiocarbonyle, (C₃-C₆)cycloalkylaminothiocarbonyle, (C₁-C₄) alkylsulfonylamino, (C₁-C₄) alkylamino, di-(C₁-C₄)alkylamino, (C₁-C₄)halogénoalkylamino, (C₃-C₆)cycloalkylamino, aminosulfonyle, (C₁-C₄)alkylaminosulfonyle, di-(C₁-C₄)alkyl-aminosulfonyle, (C₁-C₄) alkylcarbonylamino, (C₁-C₄) halogénoalkylcarbonylamino, (C₁-C₄) alkylcarbonyl-(C₁-C₂)alkyl-amino, (C₁-C₂) halogénoalkylcarbonyl-(C₁-C₂)alkyl-amino, (C₃-C₆)cycloalkylcarbonylamino, (C₃-C₆)cycloalkylcarbonyl-(C₁-C₂) alkyl-amino, (C₁-C₄)alkylthiocarbonylamino, (C₁-C₄)halogénoalkylthiocarbonylamino, (C₁-C₄) alkylthiocarbonyl-(C₁-C₂)alkyl-amino, (C₁-C₄)halogénoalkylthiocarbonyl-(C₁-C₂)alkyl-amino, (C₃-C₆)cycloalkylthiocarbonylamino ou (C₃-C₆) cycloalkylthiocarbonyl- (C₁-C₂) alkyl-amino.

3. procédé selon la revendication 1, **caractérisé en ce que**
Q représente un élément structural de la série Q2, Q3, Q4, Q10, Q11, Q13 ou Q14, où
R⁷ représente (C₁-C₄) alkyle ou (C₁-C₄) alcoxy- (C₁-C₄) alkyle et
A représente trifluorométhyle, fluoroéthyle (CH₂CFH₂, CHFCH₃), difluoroéthyle (CF₂CH₃, CH₂CHF₂, CHFCFH₂), trifluoroéthyle, (CH₂CF₃, CHFCHF₂, CF₂CFH₂), tétrafluoroéthyle(CHFCF₃, CF₂CHF₂), pentafluoroéthyle, trifluorométhylthio, trifluorométhylsulfinyle ou trifluorométhylsulfonyle,
W représente halogène ou S(O)ₙR⁸, où
R⁸ représente méthyle, éthyle, n-propyle ou isopropyle et
n représente 0, 1 ou 2,
R^{c} représente chlore,
V représente méthyle, éthyle, n-propyle ou iso-propyle et
Y représente brome, iode, cyano, éthényle, cyclopropyléthényle, i-propényle, cyclopropyléthinyle, méthyle, éthyle, isopropyle, cyclopropyléthyle, méthoxycarbonyle, trifluoroéthylaminocarbonyle, aminocarbonyle, méthylaminocarbonyle, diméthylaminocarbonyle, éthylaminocarbonyle, aminothiocarbonyle, méthylaminothiocarbonyle, diméthylaminothiocarbonyle,
ou représente cyclopropyle, cyclobutyle, cyclopentyle, cyclohexyle, cyclopentényle ou cyclohexényle à chaque fois éventuellement monosubstitué ou disubstitué, de manière identique ou différente, où les substituants peuvent à chaque fois être : méthyle, éthyle, n-propyle, i-propyle, cyclopropyle, difluorométhyle, trifluorométhyle, cyano, fluor ou chlore,
ou représente phényle, pyridin-2-yle, pyridin-3-yle, pyridin-4-yle, pyrimidin-5-yle, pyridazin-3-yle, pyridazin-4-yle, thién-2-yle, thién-3-yle, 1,3-thiazol-5-yle, 1H-imidazol-1-yle, 1H-imidazol-2-yle, 1H-imidazol-5-yle, 1H-pyrazol-1-yle, 1H-pyrazol-3-yle, 1H-pyrazol-4-yle, 1H-pyrazol-5-yle, 1H-pyrrol-1-yle, 1H-pyrrol-2-yle, 1H-pyrrol-3-yle ou 1-cyclohexényle à chaque fois éventuellement monosubstitué, disubstitué ou trisubstitué, de manière identique ou différente, où les substituants peuvent à chaque fois être : cyano, fluor, chlore, méthyle, cyclopropyle, cyanométhyle, cyanoisopropyle, cyanocylopropyle, trifluorométhyle, trifluoroéthyle ou aminocarbonyle.

4. Procédé selon la revendication 1, **caractérisé en ce que**
Q représente l'élément structural Q3 ou Q13, où
R⁷ représente méthyle ou éthyle, en particulier représente méthyle et
A représente trifluorométhyle ou pentafluoroéthyle, W représente S(O)ₙR⁸, où
R⁸ représente éthyle et
n représente 0 ou 2,
R^{c} représente chlore,
V représente méthyle ou éthyle, en particulier représente méthyle et
Y représente brome, 5-cyanopyridin-2-yle ou 6-chlorpyridazin-3-yle.

5. Procédé selon l'une quelconque des revendications 1 à 4, **caractérisé en ce que** la base organométallique au zinc est un composé de formule (VI)
(VI) (TMP)ₓ ZnCl₂₋ₓ,
dans laquelle x représente le nombre 1 ou 2.

6. Procédé selon l'une quelconque des revendications 1 à 5, **caractérisé en ce que** la base organométallique au zinc est présente en conjonction avec un halogénure alcalin ou alcalino-terreux, de préférence le chlorure de lithium et/ou le chlorure de magnésium.

7. Procédé selon l'une quelconque des revendications 1 à 6, **caractérisé en ce que** l'étape de procédé a) est réalisée à une température comprise entre 0 °C et 110 °C.

8. Procédé selon l'une quelconque des revendications 1 à 7, **caractérisé en ce que** X représente brome ou iode.

9. Procédé selon l'une quelconque des revendications 1 à 8, **caractérisé en ce que** Y représente halogène.

10. procédé selon la revendication 9, **caractérisé en ce que** le composé X-Y est un halogène élémentaire, en particulier F₂, Cl₂, Br₂ ou I₂.

11. Procédé selon l'une quelconque des revendications 1 à 8, **caractérisé en ce que** Y est défini selon l'une quelconque des revendications 1 à 4, mais ne représente pas halogène.

12. Procédé selon l'une quelconque des revendications 9 et 10, **caractérisé en ce que**
l'étape de procédé b) est réalisée à une température comprise entre 0 °C et 80 °C.

13. Procédé selon la revendication 11, **caractérisé en ce que** l'étape de procédé b) est réalisée à une température comprise entre 0 °C et 120 °C.

14. Procédé selon l'une quelconque des revendications 1 à 13, **caractérisé en ce que**
l'étape de procédé a) et l'étape de procédé b) sont réalisées en présence d'un solvant organique, le solvant étant choisi parmi le THF et le N,N-diméthylformamide (DMF) .

15. Composés de formule (IVa) 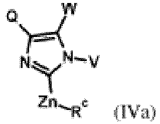 dans laquelle Q, V, R^{c} et W possèdent les significations selon l'une quelconque des revendications 1 à 4.

16. Composés de formule (IVb) 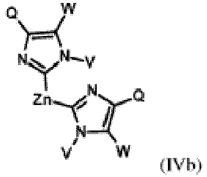 dans laquelle Q, V et W possèdent les significations selon l'une quelconque des revendications 1 à 4.

17. Composés de formule (IVa) selon la revendication 15 ou (IVb) selon la revendication 16, **caractérisé en ce que** ceux-ci sont présents de manière complexée avec des sels, les sels étant des halogénures alcalin ou alcalino-terreux, de préférence le chlorure de lithium et/ou le chlorure de magnésium.
